# EUROPEAN PATENT APPLICATION

(11) **EP 4 299 597 A1**
(43) Date of publication of application: **03.01.2024**
(21) Application number: 22758909.0
(22) Date of filing: 24.02.2022
(51) Int. Cl.: C07K 19/00, C07K 14/725, C07K 16/30, C12N 15/62, C12N 5/10, A61K 39/00, A61K 35/17, A61P 35/00, A61P 35/02

(54) **DUAL-TARGET STAR TARGETING CD19 AND CD22**

(30) Priority: 25.02.2021 CN 202110213821
(71) Applicant: Bristar Immunotech Limited, Beijing 102206 (CN)
(72) Inventor: RUI, Wei, Beijing 102206 (CN); LEI, Lei, Beijing 102206 (CN); WU, Chunyan, Beijing 102206 (CN); LIU, Fang, Beijing 102206 (CN)
(74) Representative: Michalski Hüttermann & Partner Patentanwälte mbB
(86) International application number: PCT/CN2022/077608
(87) International publication number: WO 2022/179545

(57) **Abstract**

The invention relates to the field of biomedicine, in particular to a dual-target synthetic T-cell receptor and antigen receptor (Synthetic T-Cell Receptor and Antigen Receptor, STAR) and uses thereof. Specifically, the present invention discloses a dual-target STAR targeting CD19 and CD22, T cells containing the dual-target STAR, and uses thereof.

## Description

### Technical field

The invention relates to the field of biomedicine, in particular to a dual-target synthetic T-cell receptor and antigen receptor (Synthetic T-Cell Receptor and Antigen Receptor, STAR) and uses thereof. Specifically, the present invention discloses a dual-target STAR targeting CD19 and CD22, T cells containing the dual-target STAR, and uses thereof.

### Background

Cell therapy, especially T cell-related therapy, has developed rapidly, among which chimeric antigen receptor T cell (CAR-T) therapy and TCR-T therapy have attracted much attention.

CAR-T therapy is based on the expression of CAR molecules in T cells. A CAR molecule consists of three parts: an ectodomain, which is an antigen recognition domain derived from an antibody and is responsible for recognizing a target antigen; a transmembrane domain; and an endodomain, which is a signal molecule and co-stimulatory signal molecule derived from a T cell receptor and is responsible for transducing a T cell activation signal after receiving stimulation. When CAR molecules bind to the corresponding antigens thereof, they will aggregate, initiate the effector function of T cells and kill target tumor cells.

TCR-T therapy is based on the T cell receptor (TCR). TCR is the identity of T cells, which can be divided into αβ T cells and γδ T cells based on the type of TCR. During the development, a T precursor cell will undergo VDJ rearrangement of TCR γ and TCR δ chains, which, if rearrangement is successful, will develop into a γδ T cell, or if rearrangement ends in failure, will undergo VDJ recombination of TCR α and TCR β chains, and then develop into a αβ T cell. αβ T cells account for 90%-95% of peripheral blood T cells, while γδ T cells account for 5%-10% of peripheral blood T cells. The two types of T cells recognize antigens in MHC-restricted and MHC-unrestricted ways, respectively, which play an important role in the immunity to pathogens and tumors.

The T cell receptor (TCR) complex molecule contains multiple chains, in which TCR α and TCR β chains (or TCR γ and TCR δ chains) are responsible for recognizing MHC-polypeptide molecules, and the other six CD3 subunits bind to TCR α/β chains (or TCR γ/δ chains) to play the role of signal transduction. The natural TCR complex contains ten ITAM signal sequences, which can transmit stronger signals than CAR in theory. By employing the signal transduction function of natural TCR, it is possible to construct a new receptor to alleviate T cell disability, which can play a better role against solid tumor. The ectodomain of TCR is very similar to the Fab domain of an antibody, so the variable region sequence of TCR can be replaced by a variable region sequence of an antibody, so as to obtain a Synthetic TCR and Antigen Receptor (STAR), which not only has antibody specificity, but also has superior signal transduction function of a natural TCR on mediating T cell activation.

The number of cell therapies in the field that have been put into clinical application is quite limited, so there is still a need for improved T cell therapies, such as STAR-based T cell therapies.

### Summary of the invention

In one aspect, the invention provides a synthetic T cell receptor and antigen receptor (STAR) against CD19 and CD22, which comprises an α chain comprising a first antigen binding region and a first constant region, and a β chain comprising a second antigen binding region and a second constant region,
wherein the first antigen binding region is an antigen binding region specifically binding to CD19, and the second antigen binding region is an antigen binding region specifically binding to CD22; or the first antigen binding region is an antigen binding region specifically binding to CD22, and the second antigen binding region is an antigen binding region specifically binding to CD 19.

In some embodiments, the first constant region is a modified TCR α chain constant region. In some embodiments, the modified TCRα chain constant region is derived from a mouse TCRα chain constant region, and the amino acid at position 48, such as threonine T, is mutated to cysteine C, as compared to the wild-type mouse TCRα chain constant region. In some embodiments, the modified TCR α chain constant region is derived from a mouse TCR α chain constant region, the amino acid at position 112 such as serine S is mutated to leucine L, the amino acid at position 114 such as methionine M is mutated to isoleucine I, and the amino acid at position 115 such as glycine G is mutated to valine V, as compared to the wild-type mouse TCRα chain constant region. In some embodiments, the modified TCRα chain constant region is derived from a mouse TCRα chain constant region, the amino acid at position 48, such as threonine T, is mutated to Cysteine C, the amino acid at position 112 such as serine S is mutated to leucine L, the amino acid at position 114 such as methionine M is mutated to isoleucine I, the amino acid at position 115 such as Glycine G is mutated to valine V, as compared to the wild-type mouse TCRα chain constant region. In some embodiments, the modified TCR α chain constant region is derived from the mouse TCR α chain constant region, the amino acid at position 6 such as E is substituted by D, and the amino acid K at position 13 is substituted by R, and amino acids at positions 15-18 are deleted, as compared to the wild-type mouse TCRα chain constant region. In some embodiments, the modified TCRα chain constant region is derived from the mouse TCRα chain constant region, the amino acid at position 6, such as E, is substituted by D, and the amino acid K at position 13 is substituted by R, the amino acids at positions 15-18 are deleted, the amino acid at position 48 such as threonine T is mutated to cysteine C, and the amino acid at position 112 such as serine S is mutated to leucine acid L, the amino acid at position 114 such as methionine M is mutated to isoleucine I, and the amino acid at position 115 such as glycine G is mutated to valine V, as compared to the wild-type mouse TCRα chain constant region. In some embodiments, the modified TCR α chain constant region is derived from the mouse TCR α chain constant region, and lacks the endodomain of the constant region, for example, the amino acids at positions 136-137 are deleted, as compared to the wild-type mouse TCRα chain constant region. In some embodiments, the modified TCRα chain constant region comprises an amino acid sequence shown in one of SEQ ID NOs: 3-4 and 39-41.

In some embodiments, the second constant region is a modified TCR β chain constant region. In some embodiments, the modified TCR β chain constant region is derived from the mouse TCR β chain constant region, and the amino acid at position 56, such as serine S, is mutated to cysteine C, as compared to the wild-type mouse TCRβ chain constant region. In some embodiments, the modified TCR β chain constant region is derived from the mouse TCR β chain constant region, the amino acid at position 3 such as R is substituted by K, the amino acid at position 6 such as T is substituted by F, the amino acid K at position 9 is substituted by E, S at position 11 is substituted by A, L at position 12 is substituted by V, and amino acids at positions 17 and 21-25 are deleted, as compared to the wild-type mouse TCRβ chain constant region. In some embodiments, the modified TCR β chain constant region is derived from a mouse TCR β chain constant region, the amino acid at position 56 such as serine S is mutated to cysteine C, the amino acid at position 3 such as R is substituted by K, the amino acid at position 6 such as T is substituted by F, K at position 9 is substituted by E, S at position 11 is substituted by A, and L at position 12 is substituted by V, and amino acids 17, 21-25 are deleted, a as compared to the wild-type mouse TCRβ chain constant region. In some embodiments, the modified TCR β chain constant region is derived from the mouse TCR beta chain constant region, and lacks the endodomain of the constant region, for example, the amino acids at positions 167-172 are deleted, as compared to the wild-type mouse TCRβ chain constant region. In some embodiments, the modified TCRβ chain constant region comprises an amino acid sequence shown in one of SEQ ID NOs:7-8 and 42-44.

In some embodiments, the antigen binding region is a single chain antibody (such as scFv) or a single domain antibody (such as a camelid antibody), preferably, the antigen binding region is a single chain antibody such as a scFv.

In some embodiments, the single chain antibody comprises a heavy chain variable region and a light chain variable region linked by a linker, for example, the linker is a (G4S)n linker, wherein n represents an integer from 1-10, preferably, n is 1 or 3.

In some embodiments, the antigen-binding region that specifically binds to CD19 comprises a heavy chain variable region and a light chain variable region, and the heavy chain variable region comprises VH CDR1 shown in SEQ ID NO:25, VH CDR2 shown in SEQ ID NO:26, and VH CDR3 shown in SEQ ID NO:27, the light chain variable region comprises VL CDR1 shown in SEQ ID NO:28, VL CDR2 shown in SEQ ID NO:29 , and VL CDR3 shown in SEQ ID NO:30,
for example, the antigen-binding region that specifically binds to CD 19 comprises a heavy chain variable region shown in SEQ ID NO:9, and a light chain variable region shown in SEQ ID NO: 10.

In some embodiments, the antigen-binding region that specifically binds to CD22 comprises a heavy chain variable region and a light chain variable region, wherein the heavy chain variable region comprises VH CDR1 shown in SEQ ID NO: 31, VH CDR2 shown in SEQ ID NO: 32, and VH CDR3 shown in SEQ ID NO:33, the light chain variable region comprises VL CDR1 shown in SEQ ID NO:34, VL CDR2 shown in SEQ ID NO:35 , and VL CDR3 shown in SEQ ID NO:36
for example, the antigen-binding region that specifically binds to CD22 comprises the heavy chain variable region shown in SEQ ID NO: 11, and the light chain variable region shown in SEQ ID NO: 12.

In some embodiments, the antigen binding region of the α chain or β chain is fused directly or indirectly to the N-terminus of the constant region.

In some embodiments, the antigen-binding region of the α-chain or β-chain is fused to the N-terminus of the constant region via a linker.

In some embodiments, the antigen-binding region of the α-chain or β-chain, preferably β-chain, is fused to the N-terminus of the constant region via a CD8 hinge region, for example, the CD8 hinge region comprises the amino acid sequence shown in SEQ ID NO: 13.

In some embodiments, the α chain and/or β chain has at least one exogenous intracellular functional domain linked to its C-terminus.

In some embodiments, the exogenous intracellular functional domain is linked to the C-terminus of the constant region of the α chain and/or β chain directly or through a linker, for example, the linker is a (G4S)n linker, where n represents an integer from 1-10, preferably, n is 3.

In some embodiments, the exogenous intracellular functional domain is an endodomain of a co-stimulatory molecule, preferably an endodomain of OX40, for example, the endodomain of OX40 comprises the amino acid sequence of SEQ ID NO: 14.

In some embodiments, the α chain comprises an amino acid sequence set forth in SEQ ID NO: 16, 18 or 37. In some embodiments, the β-strand comprises an amino acid sequence set forth in SEQ ID NO: 17, 19 or 38. In some embodiments, the α chain comprises the amino acid sequence set forth in SEQ ID NO:16, and the β chain comprises the amino acid sequence set forth in SEQ ID NO: 17. In some embodiments, the α chain comprises the amino acid sequence set forth in SEQ ID NO: 18, and the β chain comprises the amino acid sequence set forth in SEQ ID NO: 19. In some embodiments, the α chain comprises the amino acid sequence set forth in SEQ ID NO:37, and the β chain comprises the amino acid sequence set forth in SEQ ID NO:38.

In one aspect, the invention provides an isolated polynucleotide comprising a nucleotide sequence encoding the α-chain and/or β-chain of the STAR against CD19 and CD22 of the invention.

In one aspect, the invention provides an expression vector comprising the polynucleotide of the invention operably linked to a regulatory sequence.

In one aspect, the invention provides a method of producing a therapeutic T cell comprising expressing in the T cell the STAR against CD 19 and CD22 of the invention.

In one aspect, the invention provides a therapeutic T cell which comprises the STAR against CD19 and CD22 of the invention, or is obtained by the method of the invention.

In one aspect, the invention provides a pharmaceutical composition, which comprises the therapeutic T cell of the invention and a pharmaceutically acceptable carrier.

In one aspect, the invention provides a use of the therapeutic T cell of the invention or the pharmaceutical composition of the invention in the preparation of a medicine for the treatment of a disease in a subject, for example, the disease is a CD19 and/or CD22-related cancer.

### Brief description of the drawings

Figure 1. shows that for M971 scFv, the killing effect on CD22 target cells was significantly improved after adding CD8 hinge between it and the constant region.
Figure 2. shows that CD19-22 dual STAR (CT) also had a good killing effect on CD19 or CD22 single-target cells.
Figure 3. shows that the N-terminal modification of the constant regions of the two chains improved the killing effect of CD19-22 dual STAR.
Figure 4. Dual STAR-T cells showed significantly elevated levels of IL-2 and IFN-γ cytokines after co-culture with target cells.
Figure 5. shows that both FMC63 and M971 scFv were well expressed on the surface of T cells.
Figure 6. shows that dual STAR added with OX40 co-stimulator had stronger killing effect on CD19/CD22 target cells.
Figure 7. shows that in the mouse model, injection of single-target STAR-T cannot effectively inhibit tumor growth, while injection of dual STAR-T can effectively eliminate tumor cells.
Figure 8. shows the maps of various constructs.

### Detailed description of the invention

Unless otherwise indicated or defined, all used terms have the common meaning in the field, which will be understood by those skilled in the field. See, for example, the standard manual, such as Sambrook et al., "Molecular cloning: a laboratory manual"; Lewin, "Genes VIII", and Roitt et al., "Immunology" (8nd edition), and the general prior art cited herein; in addition, unless otherwise stated, all the methods, steps, techniques and operations that are not specifically detailed may and have been performed in a manner known per se, which will be understood by those skilled in the art. Also see, for example, the standard manual, the above general prior art and other references cited therein.

As used herein, the term "and/or" encompasses all combinations of items connected by the term, which shall be deemed to have been separately listed herein. For example, "A and/or B" covers "A", "A and B", and "B". For example, "A, B and/or C" covers "A", "B", "C", "A and B", "A and C", "B and C", and "A and B and C".

The term "comprising" is used herein to describe a sequence of a protein or nucleic acid, which may consist of said sequence, or may have additional amino acids or nucleotides at one or both ends of said protein or nucleic acid but still possess the activity described herein. In addition, those skilled in the art will understand that methionine encoded by the start codon at the N-terminal of a polypeptide is retained in certain practical situations (e.g., when expressed in a particular expression system), but does not substantially affect the function of the polypeptide. Thus, in the description of a specific polypeptide amino acid sequence, while it may not contain methionine encoded by the start codon at its N-terminal, but still covers a sequence comprising the methionine by the time, and correspondingly, the coding nucleotide sequences thereof may also contain the start codon; and vice versa.

As used herein, "the amino acid number being made reference to SEQ ID NO: x" (SEQ ID NO: x is a specific sequence listed herein) means that the position number of a particular amino acid described is the position number of the amino acid corresponding to that amino acid on SEQ ID NO: x. The amino acid correspondence between different amino acid sequences can be determined by sequence alignment methods known in the art. For example, the amino acid correspondence can be determined by an EMBL-EBI online alignment tool (https://www.ebi.ac.uk/Tools/psa/), in which two sequences can be aligned using Needleman-Wunsch algorithm with default parameters. For example, if alanine at position 46 starting from the N-terminal of a polypeptide is aligned in sequence alignment with an amino acid at position 48 of SEQ ID NO: x, then the amino acid in the polypeptide may also be described herein as "alanine at position 48 of the polypeptide, the amino acid position being made reference to SEQ ID NO: x". In the present invention, reference is made to SEQ ID NO: 2 for the amino acid position related to α chain constant region. In the present invention, reference is made to SEQ ID NO: 6 for the amino acid position related to β chain constant region.

In one aspect, the invention provides a synthetic T cell receptor and antigen receptor (STAR) against CD19 and CD22, which comprises an α chain comprising a first constant region and a β chain, comprising a second constant region,
wherein the α chain comprises an antigen binding region specifically binding to CD19, and the β chain comprises an antigen binding region specifically binding to CD22; or the α chain comprises an antigen binding region specifically binding to CD22, and the β chain comprises an antigen binding region specifically binding to CD19.

In some embodiments, the α and β chains are capable of forming a functional TCR complex upon expression in a T cell. For example, upon expression in a T cell, the α chain and β chain can combine with endogenous CD3 molecules (CD3εδ, CD3γε, CD3ζζ) to form a TCR complex of 8 subunits, and the TCR complex is displayed on the cell surface, and activates the T cell upon binding to the target antigen CD19 and/or CD22. A functional TCR complex refers to that it can activate the T cell after specifically binding to a target antigen.

In some embodiments, the first constant region is a native TCR α chain constant region, e.g., a native human TCR α chain constant region or a native mouse TCR α chain constant region. An exemplary native human TCRα chain constant region comprises the amino acid sequence shown in SEQ ID NO:1. An exemplary native mouse TCRα chain constant region comprises the amino acid sequence shown in SEQ ID NO:2.

In some embodiments, the first constant region is a modified TCR α chain constant region.

In some embodiments, the modified TCRα chain constant region is derived from the mouse TCRα chain constant region, and the amino acid at position 48, such as threonine T, is mutated to cysteine C, as compared to the wild-type mouse TCRα chain constant region.

In some embodiments, the modified TCR α chain constant region is derived from the mouse TCR α chain constant region, the amino acid at position 112 such as serine S is mutated to leucine L, the amino acid at position 114 such as methionine M is mutated to isoleucine I, and the amino acid at position 115 such as glycine G is mutated to valine V, as compared to the wild-type mouse TCRα chain constant region.

In some embodiments, the modified TCR α chain constant region is derived from the mouse TCR α chain constant region, the amino acid at position 6 such as E is substituted by D, and the amino acid K at position 13 is substituted by R, and amino acids at positions 15-18 are deleted, as compared to the wild-type mouse TCRα chain constant region.

In some embodiments, the modified TCRα chain constant region is derived from the mouse TCRα chain constant region, the amino acid at position 48, such as threonine T, is mutated to Cysteine C, the amino acid at position 112 such as serine S is mutated to leucine L, the amino acid at position 114 such as methionine M is mutated to isoleucine I, the amino acid at position 115 such as Glycine G is mutated to valine V, as compared to the wild-type mouse TCRα chain constant region.

In some embodiments, the modified TCRα chain constant region is derived from the mouse TCRα chain constant region, the amino acid at position 6, such as E, is substituted by D, and the amino acid K at position 13 is substituted by R, the amino acids at positions 15-18 are deleted, the amino acid at position 48 such as threonine T is mutated to cysteine C, and the amino acid at position 112 such as serine S is mutated to leucine acid L, the amino acid at position 114 such as methionine M is mutated to isoleucine I, and the amino acid at position 115 such as glycine G is mutated to valine V, as compared to the wild-type mouse TCRα chain constant region.

In some embodiments, the modified TCR α chain constant region is derived from the mouse TCR α chain constant region, and lacks the endodomain of the constant region, for example, the amino acids at positions 136-137 are deleted, as compared to the wild-type mouse TCRα chain constant region.

In some specific embodiments, the modified TCRα chain constant region comprises an amino acid sequence shown in one of SEQ ID NOs: 3-4 and 39-41.

In some embodiments, the second constant region is a native TCR β chain constant region, e.g., a native human TCR β chain constant region or a native mouse TCR β chain constant region. An exemplary native human TCR β chain constant region comprises the amino acid sequence shown in SEQ ID NO:5. An exemplary native mouse TCR β chain constant region comprises the amino acid sequence shown in SEQ ID NO:6.

In some embodiments, the second constant region is a modified TCR β chain constant region.

In some embodiments, the modified TCR β chain constant region is derived from the mouse TCR β chain constant region, and the amino acid at position 56, such as serine S, is mutated to cysteine C, as compared to the wild-type mouse TCRβ chain constant region.

In some embodiments, the modified TCR β chain constant region is derived from the mouse TCR β chain constant region, the amino acid at position 3 such as R is substituted by K, the amino acid at position 6 such as T is substituted by F, the amino acid K at position 9 is substituted by E, S at position 11 is substituted by A, L at position 12 is substituted by V, and amino acids at positions 17 and 21-25 are deleted, as compared to the wild-type mouse TCRβ chain constant region.

In some embodiments, the modified TCR β chain constant region is derived from a mouse TCR β chain constant region, the amino acid at position 56 such as serine S is mutated to cysteine C, the amino acid at position 3 such as R is substituted by K, the amino acid at position 6 such as T is substituted by F, K at position 9 is substituted by E, S at position 11 is substituted by A, and L at position 12 is substituted by V, and amino acids 17, 21-25 are deleted, as compared to the wild-type mouse TCRβ chain constant region.

In some embodiments, the modified TCR β chain constant region is derived from the mouse TCR beta chain constant region, and lacks the endodomain of the constant region, for example, the amino acids at positions 167-172 are deleted, as compared to the wild-type mouse TCRβ chain constant region.

In some specific embodiments, the modified TCRβ chain constant region comprises an amino acid sequence shown in one of SEQ ID NOs:7-8 and 42-44.

As used herein, "antigen-binding region" means that it alone or in combination with another antigen-binding region can specifically bind to a target antigen. In some embodiments, the antigen binding region is derived from an antibody that specifically binds to the target antigen, including any commercially available antibody.

In some embodiments, the antigen binding region may be a single chain antibody (such as scFv) or a single domain antibody (such as a camelid antibody) that specifically binds to the target antigen. In some embodiments, the antigen binding region is a single chain antibody such as a scFv.

In some embodiments, the single chain antibody comprises a heavy chain variable region and a light chain variable region linked by a linker. In some embodiments, the linker is a (G4S)n linker, wherein n represents an integer from 1-10, preferably, n is 1 or 3.

In some embodiments, the antigen-binding region that specifically binds to CD19 comprises a heavy chain variable region and a light chain variable region, and the heavy chain variable region comprises VH CDR1 shown in SEQ ID NO:25, VH CDR2 shown in SEQ ID NO:26, and VH CDR3 shown in SEQ ID NO:27, the light chain variable region comprises VL CDR1 shown in SEQ ID NO:28, VL CDR2 shown in SEQ ID NO:29 , and VL CDR3 shown in SEQ ID NO:30. In some embodiments, the antigen-binding region that specifically binds to CD19 comprises a heavy chain variable region shown in SEQ ID NO:9, and a light chain variable region shown in SEQ ID NO:10. In some embodiments, the antigen-binding region that specifically binds CD19 is a scFv, such as a scFv derived from antibody FMC63. In some embodiments, the heavy chain variable region set forth in SEQ ID NO:9 and the light chain variable region set forth in SEQ ID NO: 10 are linked by a (G4S)n linker, wherein n represents an integer from 1-10, preferably, n=3.

In some embodiments, the antigen-binding region that specifically binds to CD22 comprises a heavy chain variable region and a light chain variable region, and the heavy chain variable region comprises VH CDR1 shown in SEQ ID NO: 31, VH CDR2 shown in SEQ ID NO: 32, and VH CDR3 shown in SEQ ID NO:33, the light chain variable region comprises VL CDR1 shown in SEQ ID NO:34, VL CDR2 shown in SEQ ID NO:35 , and VL CDR3 shown in SEQ ID NO:36. In some embodiments, the antigen-binding region that specifically binds to CD22 comprises a heavy chain variable region shown in SEQ ID NO:11, and a light chain variable region shown in SEQ ID NO:12.

In some embodiments, the antigen-binding region specifically binding to CD22 comprises a heavy chain variable region and a light chain variable region, and the heavy chain variable region comprises VH CDR1 shown in SEQ ID NO:58, VH CDR2 shown in SEQ ID NO: 59, and VH CDR3 shown in SEQ ID NO:60, the light chain variable region comprises VL CDR1 shown in SEQ ID NO:55, VL CDR2 shown in SEQ ID NO:56 , and VL CDR3 shown in SEQ ID NO:57. In some embodiments, the antigen-binding region that specifically binds to CD22 comprises a heavy chain variable region shown in SEQ ID NO:46, and a light chain variable region shown in SEQ ID NO:45.

In some embodiments, the antigen-binding region specifically binding to CD22 comprises a heavy chain variable region and a light chain variable region, and the heavy chain variable region comprises VH CDR1 shown in SEQ ID NO:65, VH CDR2 shown in SEQ ID NO: 66, and VH CDR3 shown in SEQ ID NO:67, said light chain variable region comprises VL CDR1 shown in SEQ ID NO:61, VL CDR2 shown in SEQ ID NO:62 , and VL CDR3 shown in SEQ ID NO:63. In some embodiments, the antigen-binding region that specifically binds to CD22 comprises a heavy chain variable region shown in SEQ ID NO:68, and a light chain variable region shown in SEQ ID NO:64.

In some embodiments, the antigen-binding region that specifically binds to CD22 comprises a heavy chain variable region and a light chain variable region, and the heavy chain variable region comprises VH CDR1 shown in SEQ ID NO:73, VH CDR2 shown in SEQ ID NO:74, and VH CDR3 shown in SEQ ID NO:75, said light chain variable region comprises VL CDR1 shown in SEQ ID NO:69, VL CDR2 shown in SEQ ID NO:70 , and VL CDR3 shown in SEQ ID NO:71. In some embodiments, the antigen-binding region that specifically binds to CD22 comprises a heavy chain variable region shown in SEQ ID NO:76, and a light chain variable region shown in SEQ ID NO72.

In some embodiments, the antigen-binding region specifically binding to CD22 comprises a heavy chain variable region and a light chain variable region, and the heavy chain variable region comprises VH CDR1 shown in SEQ ID NO:81, VH CDR2 shown in SEQ ID NO: 82, and VH CDR3 shown in SEQ ID NO:83, said light chain variable region comprises VL CDR1 shown in SEQ ID NO:77, VL CDR2 shown in SEQ ID NO:78 , and VL CDR3 shown in SEQ ID NO:79. In some embodiments, the antigen-binding region that specifically binds to CD22 comprises a heavy chain variable region shown in SEQ ID NO:84, and a light chain variable region shown in SEQ ID NO:80.

In some embodiments, the antigen binding region that specifically binds to CD22 is a scFv, such as a scFv derived from antibodies m971, HA22, BL22, M972 or HRFB4. In some embodiments, the heavy chain variable region and the light chain variable region are linked by a (G4S)n linker, wherein n represents an integer from 1-10, preferably, n=3.

In some embodiments, the antigen binding region of the α chain or β chain is fused directly or indirectly to the N-terminus of the constant region. In some preferred embodiments, the antigen-binding region of the α-chain or β-chain, preferably β-chain, is fused to the N-terminus of the constant region via a linker. In some embodiments, the linker is a CD8 hinge region. In some embodiments, the CD8 hinge region comprises the amino acid sequence shown in SEQ ID NO:13.

In some embodiments, the α chain and/or β chain has at least one exogenous intracellular functional domain linked to its C-terminus. In some embodiments, the exogenous intracellular functional domain is linked to the C-terminus of the constant region of the α chain and/or β chain directly or through a linker. In some embodiments, the exogenous intracellular functional domain is linked to the C-terminus of the constant region of the α chain and/or β chain whose endodomain is deleted through a linker. In some embodiments, the linker is a (G4S)n linker, where n represents an integer from 1-10, preferably, n is 3.

As used herein, "exogenous" means a protein or nucleic acid sequence from a foreign species, or, if from the same species, a protein or nucleic acid sequence whose composition and/or location has been significantly altered from its native form by deliberate human intervention.

As used herein, an "exogenous intracellular domain" may be an intracellular domain of a co-stimulatory molecule such as CD40, OX40, ICOS, CD28, 4-1BB, CD27, CD137; it may also be a intracellular domain of a co-suppressive molecule, such as those of TIM3, PD1, CTLA4, LAG3; it may also be intracellular domain of cytokine receptors such as interleukin receptors (such as IL-2β receptor, IL-7α receptor or IL- 21 receptors), interferon receptors, tumor necrosis factor superfamily receptors, colony-stimulating factor receptors, chemokine receptors, growth factor receptors, or other membrane proteins; or domain of intracellular proteins such as NIK.

In some preferred embodiments, the exogenous intracellular functional domain is an endodomain of a co-stimulatory molecule, preferably an endodomain of OX40. In some embodiments, the endodomain of OX40 comprises the amino acid sequence of SEQ ID NO:14.

In some preferred embodiments, the α chain comprises an antigen-binding region specifically binding to CD19 and a modified TCR α chain constant region, wherein
the antigen-binding region specifically binding to CD19 comprises a heavy chain variable region shown in SEQ ID NO: 9 and a light chain variable region shown in SEQ ID NO: 10 linked by a linker (G4S)₃,
the modified TCR α chain constant region is derived from the mouse TCR α chain constant region, the amino acid at position 48 such as threonine T is mutated to cysteine C, the amino acid at position 112 such as serine S is mutated to leucine L, the amino acid at position 114 such as methionine M is mutated to isoleucine I, and the amino acid at position 115 such as glycine G is mutated to valine V, as compared to the wild-type mouse TCR α chain constant region.

In some preferred embodiments, the α chain comprises an antigen-binding region specifically binding to CD19 and a modified TCR α chain constant region, wherein
the antigen-binding region specifically binding to CD19 comprises a heavy chain variable region shown in SEQ ID NO: 9 and a light chain variable region shown in SEQ ID NO: 10 linked by a linker (G4S)₃,
the modified TCR α chain constant region is derived from the mouse TCR α chain constant region, the amino acid at position 6 such as E is substituted by D, K at position 13 is substituted by R, the amino acids at positions 15-18 are deleted, and the amino acid at position 48 such as threonine T is mutated to cysteine C, the amino acid at position 112 such as serine S is mutated to leucine L, and the amino acid at position 114 such as methionine M is mutated to isoleucine I, and the amino acid at position 115 such as glycine G is mutated to valine V, as compared to the wild-type mouse TCR α chain constant region.

In some preferred embodiments, the α chain comprises an antigen-binding region specifically binding to CD19, a modified TCR α chain constant region, and an endodomain of OX40, wherein
the antigen-binding region specifically binding to CD19 comprises a heavy chain variable region shown in SEQ ID NO: 9 and a light chain variable region shown in SEQ ID NO: 10 linked by a linker (G4S)₃,
the modified TCR α chain constant region is derived from the mouse TCR α chain constant region, the amino acid at position 48 such as threonine T is mutated to cysteine C, the amino acid at position 112 such as serine S is mutated to leucine L, the amino acid at position 114 such as methionine M is mutated to isoleucine I, the amino acid at position 115 such as glycine G is mutated to valine V, and the amino acids at positions 136-137 are deleted, as compared to the wild-type mouse TCR α chain constant region.

In some preferred embodiments, the α chain comprises an antigen-binding region specifically binding to CD19, a modified TCR α chain constant region, and an endodomain of OX40, wherein
the antigen-binding region specifically binding to CD19 comprises a heavy chain variable region shown in SEQ ID NO: 9 and a light chain variable region shown in SEQ ID NO: 10 linked by a linker (G4S)₃,
the modified TCR α chain constant region is derived from the mouse TCR α chain constant region, the amino acid at position 6 such as E is substituted by D, K at position 13 is substituted by R, the amino acids at positions 15-18 are deleted, and the amino acid at position 48 such as threonine T is mutated to cysteine C, the amino acid at position 112 such as serine S is mutated to leucine L, the amino acid at position 114 such as methionine M is mutated to isoleucine I, and the amino acid at position 115 such as glycine G is mutated to valine V, and amino acids 136-137 are deleted, as compared to the wild-type mouse TCR α chain constant region.

In some embodiments, the α chain comprises an amino acid sequence set forth in SEQ ID NO: 16, 18 or 37.

In some preferred embodiments, the β chain comprises an antigen-binding region specifically binding to CD22 and a modified TCR β chain constant region, wherein
the antigen-binding region specifically binding to CD22 comprises a heavy chain variable region shown in SEQ ID NO: 11 and a light chain variable region shown in SEQ ID NO: 12 linked by a linker (G4S)₃,
the modified TCRβ chain constant region is derived from the mouse TCRP chain constant region, and the amino acid at position 56 such as serine S is mutated to cysteine C, as compared to the wild type mouse TCRβ chain constant region.

In some preferred embodiments, the β chain comprises an antigen-binding region specifically binding to CD22 and a modified TCR β chain constant region, wherein
the antigen-binding region specifically binding to CD22 comprises a heavy chain variable region shown in SEQ ID NO: 11 and a light chain variable region shown in SEQ ID NO: 12 linked by a linker (G4S)₃,
the modified TCRβ chain constant region is derived from the mouse TCRβ chain constant region, and the amino acid at position 56 such as serine S is mutated to cysteine C, as compared to the wild type mouse TCRβ chain constant region,
wherein the antigen-binding region specifically binding to CD22 is linked to the modified TCRβ chain constant region through a CD8 hinge region.

In some preferred embodiments, the β chain comprises an antigen-binding region specifically binding to CD22 and a modified TCR β chain constant region, wherein
the antigen-binding region specifically binding to CD22 comprises a heavy chain variable region shown in SEQ ID NO: 11 and a light chain variable region shown in SEQ ID NO: 12 linked by a linker (G4S)₃,
the modified TCR β chain constant region is derived from the mouse TCR beta chain constant region, the amino acid at position 3, such as R, is substituted by K, and the amino acid at position 6, such as T, is substituted by F, K at position 9 is substituted by E, S at position 11 is substituted by A, L at position 12 is substituted by V, and amino acids at positions 17 and 21-25 are deleted, and the amino acid at position 56, for example, Serine S is mutated to cysteine C, as compared to the wild type mouse TCRβ chain constant region,
wherein the antigen-binding region specifically binding to CD22 is linked to the modified TCRP chain constant region through a CD8 hinge region.

In some preferred embodiments, the β chain comprises an antigen-binding region specifically binding to CD22, a modified TCR β chain constant region and an endodomain of OX40, wherein
the antigen-binding region specifically binding to CD22 comprises a heavy chain variable region shown in SEQ ID NO: 11 and a light chain variable region shown in SEQ ID NO: 12 linked by a linker (G4S)₃,
the modified TCR β chain constant region is derived from the mouse TCR beta chain constant region, the amino acid at position 3, such as R, is substituted by K, and the amino acid at position 6, such as T, is substituted by F, K at position 9 is substituted by E, S at position 11 is substituted by A, L at position 12 is substituted by V, and amino acids at positions 17 and 21-25 are deleted, and the amino acid at position 56, for example, Serine S is mutated to cysteine C, as compared to the wild type mouse TCRβ chain constant region,
wherein the antigen-binding region specifically binding to CD22 is linked to the modified TCRβ chain constant region through a CD8 hinge region.

In some embodiments, the β-strand comprises an amino acid sequence set forth in SEQ ID NO: 17, 19 or 38.

In some embodiments, the α chain comprises the amino acid sequence set forth in SEQ ID NO:16, and the β chain comprises the amino acid sequence set forth in SEQ ID NO:17. In some embodiments, the α chain comprises the amino acid sequence set forth in SEQ ID NO:18, and the β chain comprises the amino acid sequence set forth in SEQ ID NO:19. In some embodiments, the α chain comprises the amino acid sequence set forth in SEQ ID NO:37, and the β chain comprises the amino acid sequence set forth in SEQ ID NO:38.

In some embodiments, the α chain comprises the amino acid sequence set forth in SEQ ID NO:48, and the β chain comprises the amino acid sequence set forth in SEQ ID NO:47. In some embodiments, the α chain comprises the amino acid sequence set forth in SEQ ID NO:50, and the β chain comprises the amino acid sequence set forth in SEQ ID NO:49. In some embodiments, the α chain comprises the amino acid sequence set forth in SEQ ID NO:52, and the β chain comprises the amino acid sequence set forth in SEQ ID NO:51. In some embodiments, the α chain comprises the amino acid sequence set forth in SEQ ID NO:54, and the β chain comprises the amino acid sequence set forth in SEQ ID NO:53.

In another aspect, the present invention provides an isolated polynucleotide comprising a nucleotide sequence encoding the α-chain and/or β-chain of the synthetic T cell receptor and antigen receptor (STAR) of the present invention.

In some embodiments, the polynucleotide comprises i) a nucleotide sequence encoding the α chain, ii) a nucleotide sequence encoding the β chain, and iii) a nucleotide sequence encoding a self-cleavage peptide located between i) and ii), in a same reading frame. The nucleotide sequence encoding the α chain may be located at the 5' end or the 3' end of the nucleotide sequence encoding the β chain.

As used herein, the "self-cleavage peptide" means a peptide that can achieve self-cleavage in a cell. For example, the self-cleavage peptide may contain a protease recognition site so as to be recognized and specifically cleaved by proteases in a cell.

Alternatively, the self-cleavage peptide may be a 2A polypeptide. The 2A polypeptide is a kind of short peptide from virus, and its self-cleavage occurs during translation. When two different target proteins are linked by 2A polypeptide and expressed in the same reading frame, the two target proteins are generated almost in a ratio of 1:1. A common 2A polypeptide may be P2A from porcine techovirus-1, T2A from Thosea asigna virus, E2A from equine rhinitis A virus, and F2A from foot-and-mouth disease virus. Among them, P2A has the highest cleavage efficiency and is therefore preferred. A variety of functional variants of these 2A polypeptides are also known in the art, which can also be used in the present invention.

In another aspect, the present invention provides an expression vector comprising the polynucleotide of the present invention operably linked to a regulatory sequence.

The "expression vector" of the present invention may be a linear nucleic acid fragment, a cyclic plasmid, a viral vector, or an RNA capable of translation (e.g., mRNA). In some preferred embodiments, the expression vector is a viral vector, such as a lentiviral vector.

The term "regulatory sequence" and "regulatory element" are used interchangeably to refer to a nucleotide sequence that is located upstream (5' non-coding sequence), intermediate or downstream (3' non-coding sequence) of a coding sequence and affect the transcription, RNA processing or stability, or translation of the relevant coding sequence. An expression regulatory element refers to a nucleotide sequence that can control the transcription, RNA processing or stability, or translation of a nucleotide sequence of interest. A regulatory sequence may include, but is not limited to, a promoter, a translation leader sequence, an intron, an enhancer, and a polyadenylation recognition sequence.

As used herein, the term "operably linked" means that a regulatory element (e.g., but not limited to, a promoter sequence, a transcription termination sequence, etc.) is linked to a nucleic acid sequence (e.g., a coding sequence or an open reading frame) such that the nucleotide sequence transcription is controlled and regulated by the transcriptional regulatory element. Techniques for operably linking a regulatory element region to a nucleic acid molecule are known in the art.

In another aspect, the present invention provides a method of producing a therapeutic T cell comprising expressing in the T cell the synthetic T cell receptor antigen receptor (STAR) of the present invention. In some embodiments, the method comprises introducing the polynucleotide of the invention or the expression vector of the invention into the T cell.

In another aspect, the invention provides a therapeutic T cell which comprises the synthetic T cell receptor antigen receptor (STAR) of the invention, or is obtained by the method of the invention.

The T cell of the present invention may be obtained by various non-limiting methods from a number of non-limiting sources, including peripheral blood mononuclear cells, bone marrow, lymph node tissue, cord blood, thymus tissue, ascite, pleural effusion, spleen tissue, and tumor. In some embodiments, the cell may be derived from a healthy donor or from a patient diagnosed as cancer. In some embodiments, the cell may be part of a mixed population of cells showing distinct phenotypic profiles. For example, T cells can be obtained by isolating peripheral blood mononuclear cells (PBMC), then activating and amplifying with a specific antibody.

In some embodiments of aspects of the invention, the T cells are derived from autologous cells of a subject. As used herein, "autologous" means that cells, cell line, or population of cells used to treat a subject is derived from the subject. In some embodiments, the immune cells, such as T cells, are derived from allogeneic cells, such as a donor compatible with the subject human leukocyte antigen (HLA). Cells from donors can be converted into non-alloreactive cells using standard protocols and replicated as needed to produce cells that can be administered to one or more patients.

In another aspect, the present invention provides a pharmaceutical composition, which comprises the therapeutic T cell of the present invention and a pharmaceutically acceptable carrier.

As used herein, a "pharmaceutically acceptable carrier" includes any and all physiologically compatible solvents, dispersion medium, coatings, antibacterial and antifungal agents, isotonic agents and absorption retarders, etc. Preferably, the carrier is suitable for intravenous, intramuscular, subcutaneous, parenteral, spinal, or epidermal administration (e.g., by injection or infusion).

In another aspect, the present invention provides the use of the therapeutic T cell of the present invention in the preparation of a medicine for the treatment of a disease such as cancer in a subject.

As use herein, "subject" refers to an organism that suffers from or is prone to suffer from a disease (e.g., cancer) that can be treated by the cell, method, or pharmaceutical composition of the present invention. A non-limiting example includes human, cattle, rat, mouse, dog, monkey, goat, sheep, cow, deer, and other non-mammals. In some preferred embodiments, the subject is human.

In another aspect, the present invention provides a method for treating a disease such as cancer in a subject, the method comprising administering to the subject an effective amount of therapeutic immune cell or pharmaceutical composition of the present invention.

As used herein, a "therapeutically effective amount" or "therapeutically effective dose" or "effective amount" refers to the amount of a substance, compound, material or cell that is at least sufficient to produce a therapeutic effect after administration to a subject. Therefore, it is an amount necessary to prevent, cure, improve, block or partially block the symptoms of disease or disorder. For example, an "effective amount" of the cell or pharmaceutical composition of the present invention may preferably result in a decrease in the severity of disorder symptoms, an increase in the frequency and duration of the asymptomatic period of the disorder, or the prevention of injury or disability as a result of suffering from the disorder. For example, for the treatment of tumor, an "effective amount" of the cell or pharmaceutical composition of the present invention may preferably inhibit tumor cell growth or tumor growth by at least about 10%, preferably at least about 20%, more preferably at least about 30%, more preferably at least about 40%, more preferably at least about 50%, more preferably at least about 60%, more preferably at least about 70%, and more preferably at least about 80%, as compared to an untreated subject. The ability to inhibit tumor growth can be evaluated in an animal model system that may predict efficacy in a human tumor. Alternatively, it is possible to perform evaluation by examining the ability to inhibit the growth of tumor cells which may be determined in vitro by tests known to those skilled in the art.

In practice, the dose level of cells in the pharmaceutical composition of the present invention may vary to obtain an amount of the active ingredient that can effectively achieve the desired therapeutic response to a specific patient, composition and administration route without toxicity to the patient. The chosen dose level depends on a variety of pharmacokinetic factors, including the activity of the applied particular composition of the invention, administration route, administration time, excretion rate of the applied particular compound , duration of treatment, applied other drugs, compounds and/or materials in combination with the applied particular composition, age, gender, weight, condition, general health and medical history of the patient to be treated, and similar factors known in the medical field.

The administration of the therapeutic T cell or pharmaceutical composition or drug according to the present invention may be carried out in any convenient manner, such as through injection, infusion, implantation or transplantation. The administration of the cell or composition described herein may be intravenous, intralymphatic, intradermal, intratumoral, intramedullary, intramuscular, or intraperitoneal administration. In one embodiment, the cell or composition of the present invention is preferably administered by intravenous injection.

In embodiments of various aspects of the invention, the disease is a CD19 and/or CD22-related disease, such as a CD19 and/or CD22 abnormal expression-related disease, such as a CD19 and/or CD22-related cancer. The cancer may be a B-cell malignant tumor, such as chronic or acute leukemia (including acute myeloid leukemia, chronic myeloid leukemia, acute lymphoblastic leukemia, chronic lymphoblastic leukemia), lymphocytic lymphoma, non-Hodgkin's lymphoma, and combinations thereof. In some embodiments, the cancer is a CD19 and/or CD22 positive cancer.

### Example

### Experimental materials and methods

### Vector construction

The lentiviral vectors and lentiviral packaging plasmids used in the examples of this application were purchased from commercial companies or synthesized by commercial companies. The gene fragments used in the examples of this application, including signal peptides, antibody binding regions, hinge regions, TCR constant regions, tag proteins, etc., were synthesized from commercial companies. By means of PCR with synthetic primers, one or more target fragments were linked to obtain corresponding functional sequences. The lentiviral vector used in the present invention is pHAGE-hEF1α-RFP. The pHAGE-hEF1A-WPRE-AMP vector was obtained using restriction endonuclease SpeI/SalI. The fragment genes were obtained by synthesis and PCR method. Then the complete vector was obtained by homologous recombination method under the action of recombinase.

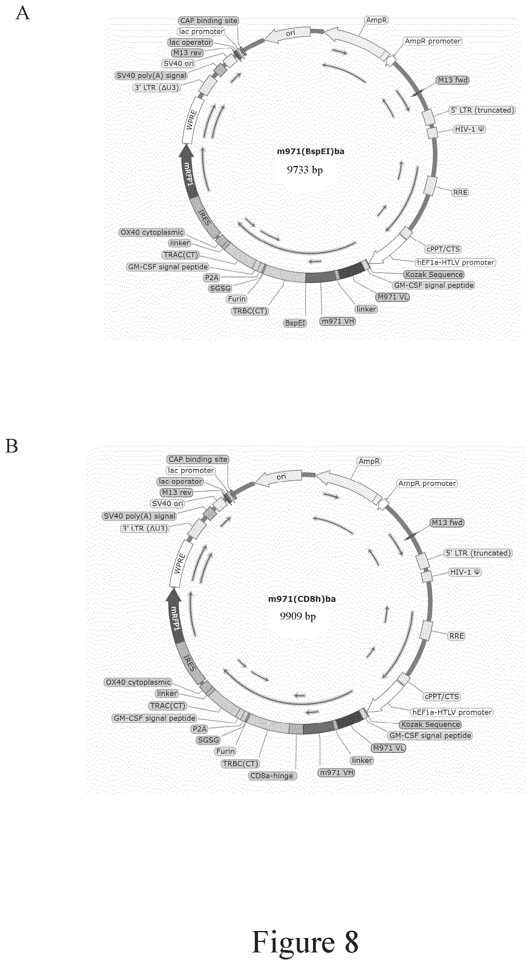

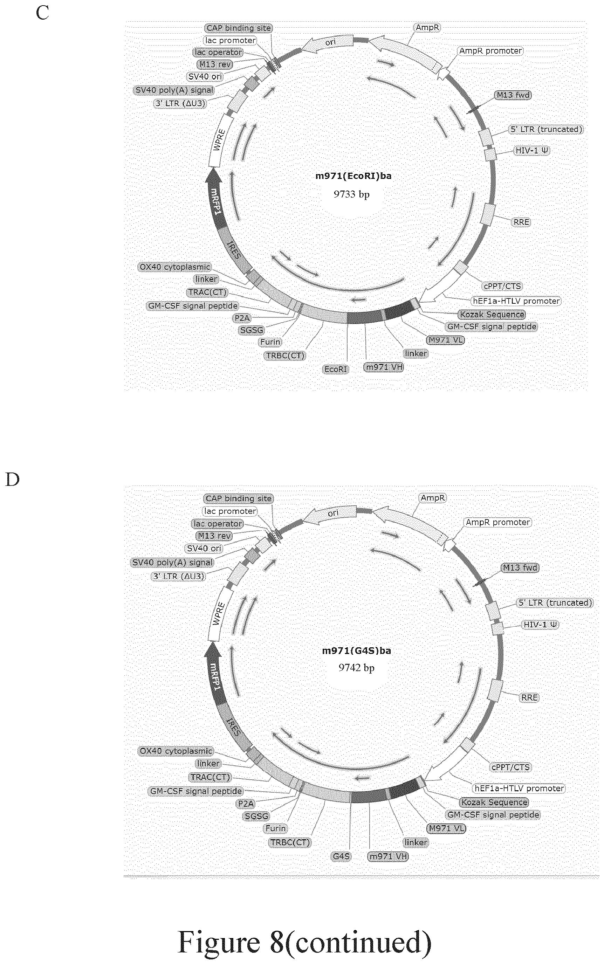

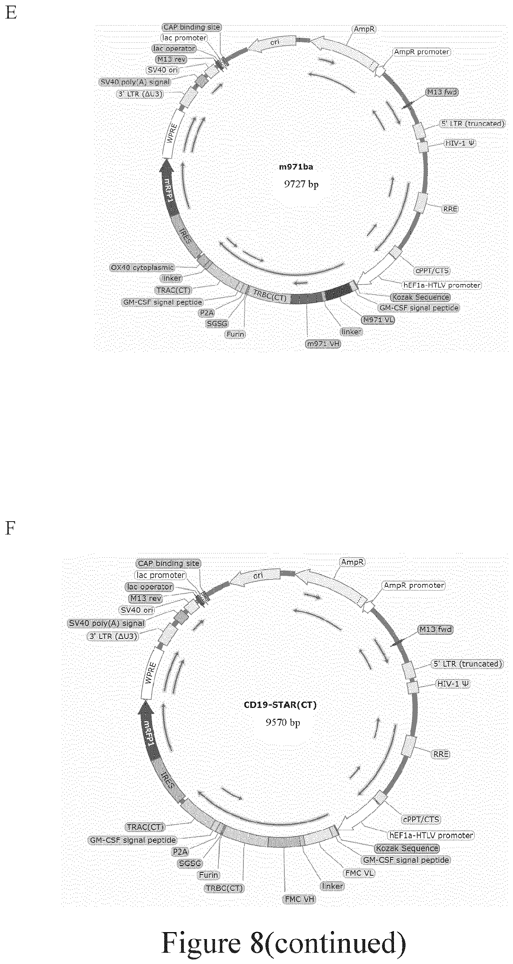

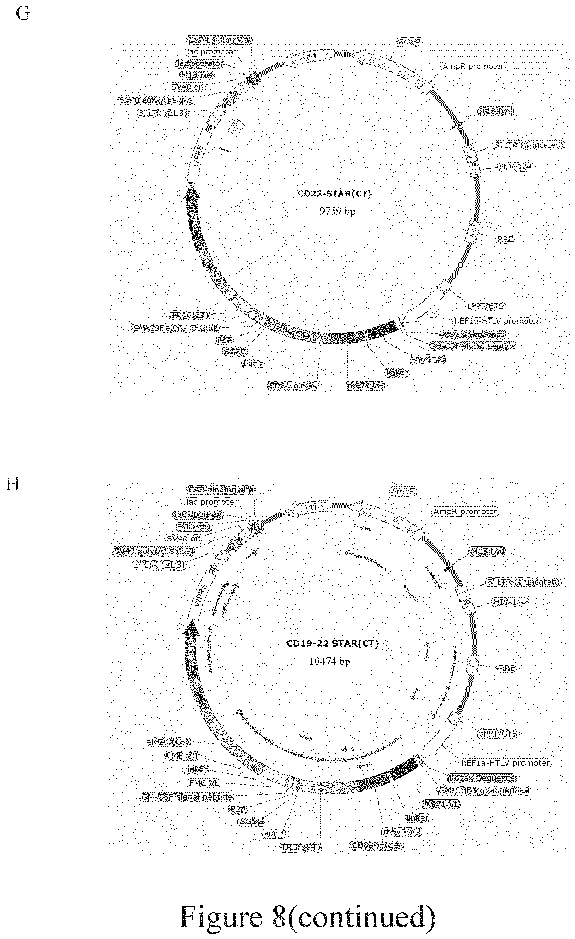

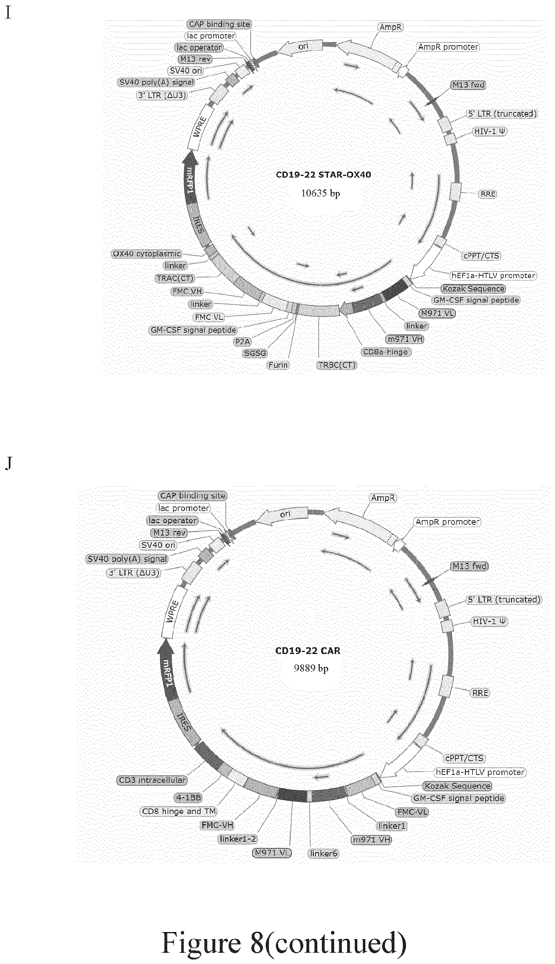

The following constructs were constructed in the examples of this application: CD19-22 CAR, CD19-22 STAR(CT), CD19-22 STAR-OX40, CD19-STAR(CT), CD22-STAR(CT), M971(BspEI)ba , M971(CD8h)ba, M971(EcoRI)ba, M971(G4S)ba, M971ba. Maps of the constructs are shown in Figure 8.

The amino acid sequence of CD19-22 CAR as a control is shown in SEQ ID NO:24.

The scFv of M971 was selected as the antigen-binding region targeting CD22. The amino acid sequence of M971 heavy chain variable region (VH) is shown in SEQ ID NO: 11; the amino acid sequence of the light chain variable region (VL) is shown in SEQ ID NO: 12. The scFv of FMC63 was selected for the antigen-binding region targeting CD19. The amino acid sequence of the FMC63 heavy chain variable region (VH) is shown in SEQ ID NO:9; the amino acid sequence of the light chain variable region (VL) is shown in SEQ ID NO: 10. The linker connecting VH and VL is GGGGSGGGGSGGGGS (i.e. (G4S)₃). The amino acid sequence of the CD8 hinge region is shown in SEQ ID NO: 13.

The constant region of the α chain is derived from mouse, the wild type is named TRAC, and the sequence is shown in SEQ ID NO:2. TRAC(CT) refers to the murine constant region with the mutation of threonine T at position 48 to cysteine C and the mutation from LSVMGLRIL to LLVIVLRIL. Its amino acid sequence is shown in SEQ ID NO:3. TRAC(NCT) refers to the inclusion of N-terminal modification based on TRAC(CT). The amino acid sequence of TRAC(NCT) is shown in SEQ ID NO:4.

The constant region of the β chain is derived from mouse, the wild type is named TRBC, and the sequence is shown in SEQ ID NO:6. TRBC(CT) refers to the murine constant region in which serine S at position 56 is mutated to cysteine C, and its amino acid sequence is shown in SEQ ID NO:7. TRBC(NCT) refers to the inclusion of N-terminal modification based on TRBC(CT). The amino acid sequence of TRBC(NCT) is shown in SEQ ID NO:8.

The amino acid sequence of the cytoplasmic region of costimulatory molecule OX40 is shown in SEQ ID NO:14.

### Lentiviral packaging

Lentix-293T cells were inoculated into 10 cm culture dishes at 5×10⁵ cells/mL, cultured in a 37°C, 5% CO₂ incubator, and transfected when the cell density reached about 80% (observed under a microscope). The four plasmids were mixed evenly with 500uL serum-free DMEM according to the ratio of PMD2.G:REV:PMDLG:transfer plamid=1:1:2:4. 54uL PEI-max was mixed with 500uL serum-free DMEM evenly, and placed at room temperature for 5min (the volume-mass ratio of PEI-Max and plasmid was 3:1). The PEI-max mixture was slowly added to the plasmid mixture, gently pipetted, mixed well, and placed at room temperature for 15 minutes. The final mixture was slowly added into the culture medium, mixed well, put back into the incubator for culturing for 12h-16h, then changed to 6% FBS DMEM medium for culturing, and the virus liquid was collected at 48 h and 72 h.

### Culture and infection of T cells

### 1) Culture of Jurkat T cell line

The Jurkat T cell line was cultured in RPMI 1640 medium containing 10% FBS. The culture density was 3*10⁵/ml, with the highest density not more than 3*10⁶/ml. The cells were subcultured every 1-2 days. The required amount of cells were taken after counting the cells, and the medium was added to adjust to the above density, and cultured in a CO₂ incubator .

### 2) Infection of Jurkat T cell line

After cell counting, 1*10⁶/ml cells were centrifuged to replace the medium, resuspended with 1ml RPMI 1640 medium containing 10% FBS, added to a 24-well plate, and virus solution was added (MOI=1), centrifuged at 1500 rpm for 90 min, placed in a CO₂ incubator. After 12 hours of infection, the medium was completely changed to fresh RPMI 1640 medium containing 10% FBS, and the positive rate was detected within 72 hours.

### 3) Culture of human primary T cells

After the primary T cells were obtained by Ficoll separation method, they were cultured in X-VIVO medium containing 10% FBS and 100 IU/ml IL-2. The initial culture density was 1*10⁶/ml. The cells were added to a CD3 and RetroNectin (final concentration 5 µg/ml) pre-coated plate. During the later stage, the culture density was 5*10⁵/ml, with the highest not more than 3*10⁶/ml, and the cells were subcultured every 1-2 days.

### 4) Infection of human primary T cells

After the primary T cells were cultured for 48 hours, the virus solution was added, MOI=20, centrifuged at 1500 rpm for 90 minutes, and placed in a CO₂ incubator for culture. After 24 hours of infection, X-VIVO medium containing 10% FBS and 100 IU/ml IL-2 was supplemented and transferred to another well, and the infection efficiency was detected by tag protein or antibody at 72 hours.

### 5) Detection of infection efficiency

After 72 hours of infection, the cells were blown evenly and counted, and 5*10⁵/ml was taken for centrifugation, and the supernatant was discarded. The staining solution was PBS+2% FBS+2 mM EDTA, and the corresponding antibody was added, incubated for 30 minutes, and then washed twice with PBS, detected by the machine.

### Test of the in vitro function of STAR-T cells

Positive T cells were co-cultured with Luciferase-expressing target cells (Raji cells, CD19-KO Raji cells or CD22-KO Raji cells) at specified effect-to-target ratios.

After co-cultivation for 24 h, the cell suspension was gently blown evenly, and 150 µL of the cell suspension of each well was added to a white 96-well plate, each with two replicate wells, and luciferase substrate was added. The mixture was shaken (at low speed) and incubated for 10 min, then a multifunctional microplate reader was used to detect the chemiluminescence value. Calculation of cell killing: killing efficiency=100%-(value of the effector cell-target cell well / value of the control cell-target cell well). Alternatively, after 24 hours of co-cultivation, the cell culture supernatant was collected, and the content of cytokines was detected by a commercial kit.

### Test of the in vivo function of STAR-T cells

The model was constructed using NSG immunodeficient mice. The mouse genotype is NOD-Prkdcem26Il2rgem26/Nju, lacking T cells, B cells, NK cells, and also defective for macrophages and dendritic cells. In this experiment, female NSG mice aged 6-8 weeks were used, and the weight difference of mice in each batch of experiments was controlled within 2 g. Mice were housed in specific pathogen-free (SPF) individually ventilated cages, provided with a normal diet and drinking water with an acidic pH to prevent pathogen contamination.

Raji cell, a human Burkitt's lymphoma cell line, was used to construct tumor model by xenografting. Raji cell is a cell line expressing the luciferase gene through lentiviral vectors, and the development and changes of Raji tumors may be monitored in real time by means of luciferin chemiluminescence and in vivo imaging in mice. In this model, 1-3×10⁶ Raji-luciferase cells were inoculated into 6-8-week-old female NSG mice through tail vein reinfusion. By intraperitoneally injecting fluorescein potassium salt solution into mice, the fluorescent signal of tumor cells in vivo was detected by live imaging.

### Example 1. STAR structure and optimization

### 1) STAR design

The secreted antibody (Antibody, Ab) or B-cell receptor (BCR) produced by B cells has great similarity to the T-cell receptor (TCR) in terms of gene structure, protein structure and spatial conformation. Both the antibody and TCR consist of a variable region and a constant region, in which the variable region plays the role of antigen-recognizing and binding, while the constant region domain plays the role of structural interaction and signal transduction. By replacing the variable regions of TCR α and β chains (or TCR γ and δ chains) with the heavy chain variable region (VH) and light chain variable region (VL) of an antibody, an artificially synthetic chimeric molecule called Synthetic T-Cell Receptor and Antibody Receptor (STAR) can be constructed.

A STAR molecule has two chains, wherein the first chain is obtained by fusing an antigen recognition sequence (such as an antibody heavy chain variable region) with the constant region (C α) of a T cell receptor α chain (TCR α), and the second chain is obtained by fusing an antigen recognition sequence (such as an antibody light chain variable region) with the constant region (C β) of a T cell receptor β chain (TCR β). The antigen recognition domain (such as VH, VL or scFv) and the constant domain (constant region of TCR α, β) in the construct can be arranged and combined to form a variety of constructs with different configurations but similar functions.

The first and second chains of STAR molecule, after expressing in T cells, will combine with endogenous CD3 ε δ, CD3 γ ε and CD3 ζ chains in the endoplasmic reticulum to form a eight-subunit complex, which is present on the surface of cell membrane in the form of complex. An immunoreceptor tyrosine-based activation motif (ITAM) is a signal transduction motif in a TCR molecule, with its conserved sequence of YxxL/V. The endodomains of CD3 ε, δ, γ and ε chains comprise one ITAM sequence, and that of CD3 ζ chain comprises three ITAM sequences, so a complete STAR complex has a total of ten ITAM sequences. When the antigen recognition sequence of a STAR receptor binds to its specific antigen, the intracellular ITAM sequence will be phosphorylated successively, which then in turn activate the downstream signaling pathway, activating transcription factors such as NF-κ β, NFAT, and AP-1, to initiate the activation of T cells and produce effector functions.

### 2) STAR optimization

STAR is a structure designed based on TCR, and because T cells themselves express TCR, STAR can mismatch with endogenous TCR, resulting in new structures without function. In the present invention, murineization of the constant region, cysteine point mutation, N-terminal modification, and hydrophobic amino acid mutation of the constant region of the α chain improve the function of the STAR.

Constant region murinization modification: since the constant region sequences of human, primate and mouse TCRα/β chains have high functional conservation and the key amino acid sequences are the same, they can be exchanged with each other. After the exchange, on the one hand, the efficiency of correct pairing of STAR molecules is increased, and on the other hand, the possibility of unknown specificity caused by mismatching is reduced, which increases safety.

Cysteine point mutation to introduce disulfide bond: threonine T at position 48 in the constant region of the murineized TCRα chain was mutated to cysteine C, and serine S at position 56 in the constant region of the murineized TCRβ chain was mutated to cysteine C. These two newly added cysteines would form a disulfide bond between the two chains of STAR, reduce the mismatch between the two chains of STAR and the endogenous TCR chain, and help STAR molecules form a more stable complex.

The design of hydrophobic amino acid substitution in the transmembrane region of STAR: three amino acid sites in the transmembrane region of the constant region of the TCRα chain from 111 to 119 were mutated, serine S at position 112 mutated to leucine L and Methionine M at position 114mutated to isoleucine I, glycine G at position 115 mutated to valine V. The overall amino acid sequence of this region was mutated from LSVMGLRIL to LLVIVLRIL. This design increases the hydrophobicity of the transmembrane region, reduces the instability caused by the positive charge carried by the TCR transmembrane region, and enables STAR molecules to exist more stably on the cell membrane, thereby obtaining better functions.

In order to further optimize the design of the STAR molecule, specific rearrangement of the N-terminus of the constant region of the STAR molecule was carried out on the basis of murineization of the constant region, cysteine point mutation and hydrophobic amino acid mutation of the α-chain constant region to obtain better effects. Rearrangement means to delete part of the sequence and at the same time carry out humanization mutation on part of the sequence. The significance of humanized mutations is to reduce the non-human sequences in STAR molecules as much as possible while ensuring the function of STAR molecules, so as to avoid the possibility of STAR-T cells being rejected by receptors in clinical applications to the greatest extent.

The rearrangement scheme of 18 amino acids at the N-terminal of the constant region of the TCRα chain (the mouse sequence is DIQNPEPAVYQLKDPRSQ): through the analysis of the amino acid properties, it was found that the amino acid substitutions at the E6D, K13R, R16K, and Q18S sites of the mouse and human sequences were of the same properties. However, for the P15S site, non-polar amino acid was replaced by a polar amino acid, so it can be considered that the properties of the protein near this site are not conserved and can be modified without affecting the function. In summary, the sequence of amino acids 1-14 was retained and humanized, and the sequence of amino acids 15-18 was deleted.

The rearrangement scheme of the 25 amino acids at the N-terminal of the constant region of the TCRβ chain (the mouse sequence is DLRNVTPPKVSLFEPSKAEIANKQK): through the analysis of amino acid properties, it was found that only the R3K and L12V sites between the mouse and human sequences belonged to amino acid substitutions of the same properties, while the T6F, K8E The , S11A, K17E, A21S, N22H and K23T sites are all substitutions with amino acids of different properties, so it can be considered that the protein properties near these sites are not conserved, and can be modified without affecting the function. In summary, the sequence of amino acids 1-16 was retained and humanized, and the amino acids 17, 21-25 were deleted.

In addition, the effect of an additional hinge structure on STAR was also tested. Specifically, the scFv of M971 targeting CD22 was constructed at the N-terminal of the β chain constant region of STAR, and different hinge structures were added therebetween (respectively, the amino acid sequence encoded by BspEI, the amino acid sequence encoded by EcoRI, the hinge region of CD8, G4S linker). Constructs M971(BspEI)ba, M971(CD8h)ba, M971(EcoRI)ba, M971(G4S)ba, M971ba were obtained. In these constructs, the C-terminus of the α-chain constant region (the endodomain deleted) was linked to the endodomain of the co-stimulatory molecule OX40 via a linker (G4S)₃. The resulting constructs were transduced into T cells to obtain corresponding STAR-T cells. The specific killing of different STAR-T cells obtained on CD22-expressing Raji cells was tested (the effect-to-target ratios of 0.5:1 and 1:1 were used, respectively).

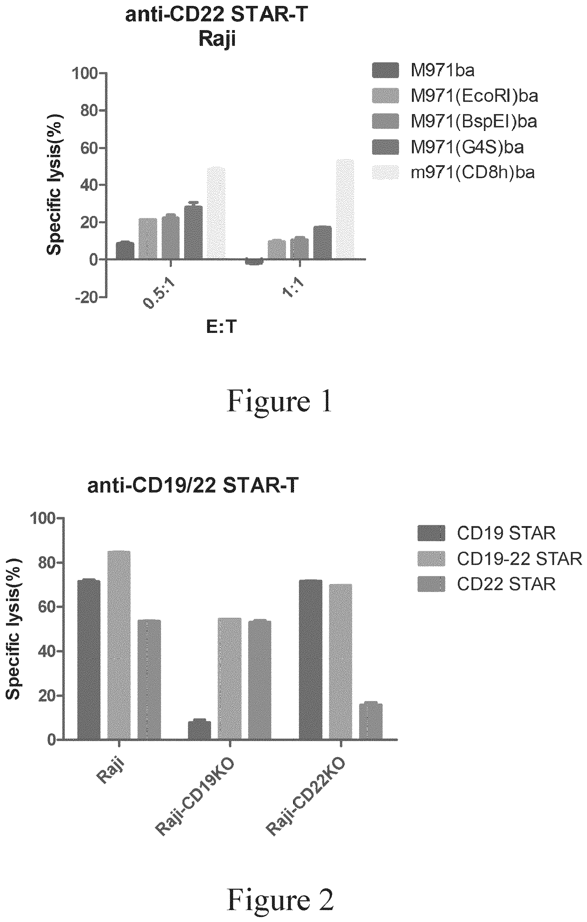

The experimental results are shown in Figure 1. It was found that for the STAR using the M971 scFv against CD22, the killing effect on CD22 target cells after adding the CD8 hinge was significantly better than other structures.

### Example 2. Dual-target STAR targeting CD19 and CD22

A CD19-targeting STAR was constructed using the FMC63 scFv targeting CD19, wherein the FMC63 scFv was constructed at the N-terminal of the β-chain constant region, and the STAR structure was named CD19-STAR (CT). CT represents the murine constant region with the cysteine modification (C) and the hydrophobic modification (T) of the α-chain transmembrane region described in Example 1.

A CD22-targeting STAR was constructed using the M971 scFv targeting CD22, wherein the M971 scFv was constructed at the N-terminal of the β-chain constant region, and the STAR structure was named CD22-STAR (CT).

A dual-target STAR targeting both CD19 and CD22, namely CD19-22 STAR (CT), was constructed using FMC63 scFv against CD19 and M971 scFv against CD22, wherein M971 scFv was constructed at the N-terminal of the constant region of the β-chain, and FMC63 scFv was constructed at the N-terminal of the α-chain.

Then, CD19 or CD22 knockout Raji cell line was constructed using the CRISPR-Cas9 system for detecting the killing effect of CD19-22 STAR (CT) on a single target, and CD19-STAR (CT) and CD22-STAR (CT) were used as control. The results are shown in Figure 2. It can be seen that CD19-22 STAR (CT) also has a good killing effect on CD19 and CD22 single-target cells.

In order to further detect the specific killing of M971 and FMC63 scFv on CD22/19 targets, the human kidney epithelial cell line 293T that does not express CD19 and CD22 was used as target cells. 293T cells are cells overexpressing luciferase-GFP by lentivirus. At the same time, 293T-CD19/22 target cells overexpressing CD19 or CD22 were constructed by lentivirus on the basis of these cells. The results showed that CD19-22 STAR (CT) basically did not kill 293T cells, but could kill 293T target cells overexpressing CD19 or CD22.

In addition, another HA22 scFv targeting CD22 was used instead of M971 scFv, combined with FMC63 scFv to construct a dual-target STAR targeting both CD19 and CD22, and the CD8 hinge region was also used to connect the HA22 scFv and the N-terminal of β-chain constant region. The obtained dual-target STAR also showed strong killing ability to 293T cells overexpressing CD19 and CD22, although the killing ability and specificity were weaker than the M971 scFv-based dual-target STAR. The efficacy of HA22 scFv-based dual-target STARs can be improved by optimizing the hinge region connecting the HA22 scFv and β-chain constant region.

### Example 3. Optimizing CD19-22 STAR by modifying the N-terminal of the constant region

In order to further improve the CD19-22 STAR, its structure was optimized, especially on the basis of CD19-22 STAR (CT), the N-termini of the constant regions of the α chain and β chain were further modified as described in Example 1 to obtain CD19-22 STAR (NCT): α chain sequence is shown in SEQ ID NO:37; β chain sequence is shown in SEQ ID NO:38. The N in NCT stands for N-terminal modification. Then the killing ability of CD19-22 STAR (CT), CD19-22 STAR (NCT) and CD19-22 CAR as a control was tested against lymphoma cell line Raji cells, CD19 knockout Raji cells, CD22 knockout Raji cells.

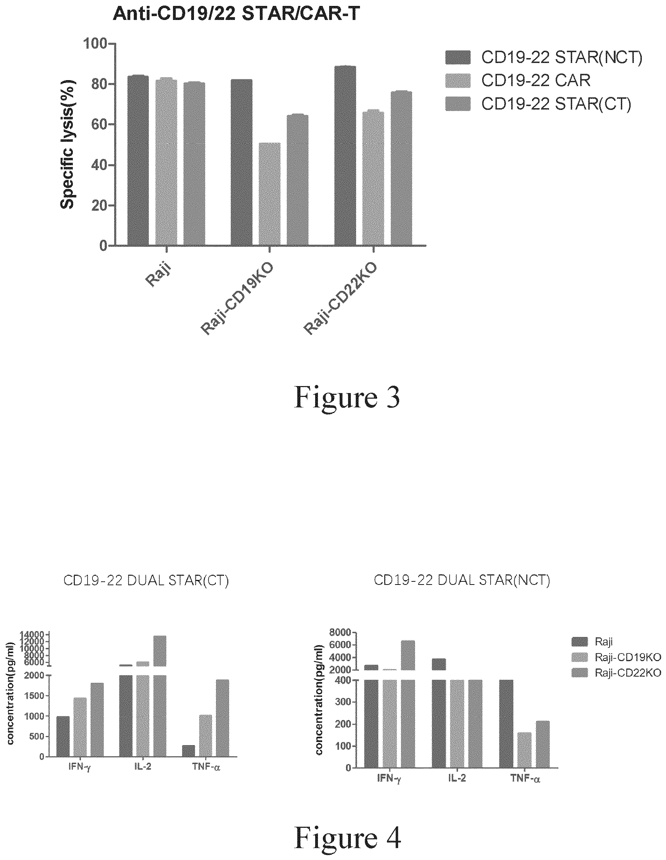

The experimental results are shown in Figure 3. It was found that CD19-22 STAR (NCT) has better killing effect on CD19/CD22 single-target cells than N-terminal unmodified CD19-22 STAR (CT) and CD 19-22 CAR.

In addition, the effect of dual STAR on cytokine secretion was also detected. Two kinds of CD19-22 STAR-T cells were co-cultured with target cells for 24 hours, and the culture supernatant was collected for detecting cytokines IL-2, IFN-γ and TNF-α. As shown in Fig. 4, the levels of IL-2 and IFN-γ cytokines were significantly increased, while the level of TNF-α was relatively low.

### Example 4. Optimizing CD19-22 STAR by adding co-stimulatory factors

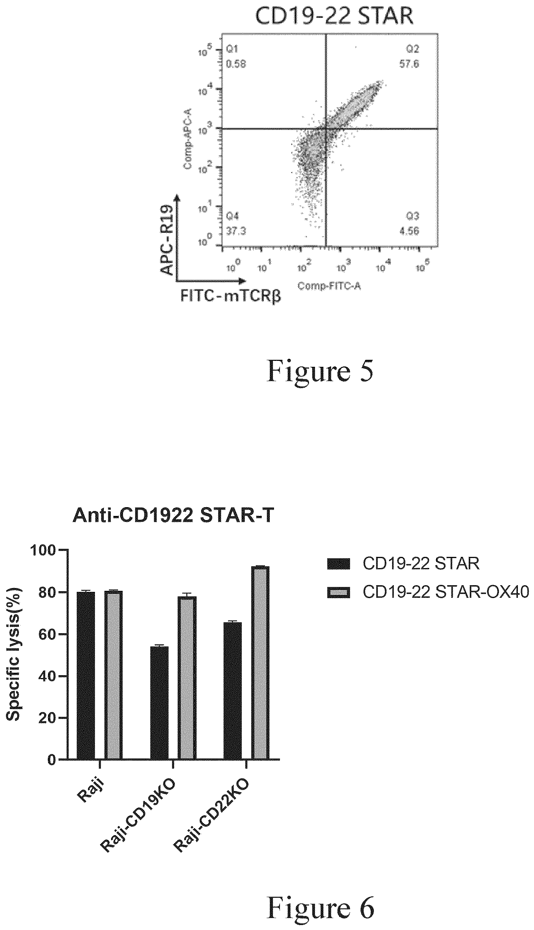

The CD19-22 STAR (CT) structure was further optimized by deleting the C-terminal natural intracellular region in the α-chain (for the α-chain constant region derived from mouse, the last 2 amino acids SS of the C-terminus), and OX40 co-stimulatory factor was added through the linker (G4S)₃ to obtain CD19-22 STAR-OX40 (α chain: SEQ ID NO: 18; β chain: SEQ ID NO: 19) and its killing ability was tested. After transfecting T cells with the CD19-22 STAR-OX40 construct, R19 (Anti-Mouse FMC63 scFv mAb (R19M)) that recognizes FMC63, and mTCRb (M971 scFv constructed at the N-terminal of the β chain) that recognizes the mouse TCR β chain was used for flow cytometry analysis. The results are shown in Figure 5. Both FMC63 and mTCRb can be expressed on the surface of T cells, and the expression is basically the same, indicating that both FMC63 and M971 scFv can be well presented on the membrane.

The results on target cells are shown in Figure 6. CD19-22 STAR with OX40 co-stimulator has stronger killing effect on CD19/CD22 target cells.

### Example 5. In vivo function detection of CD19-22 STAR-OX40

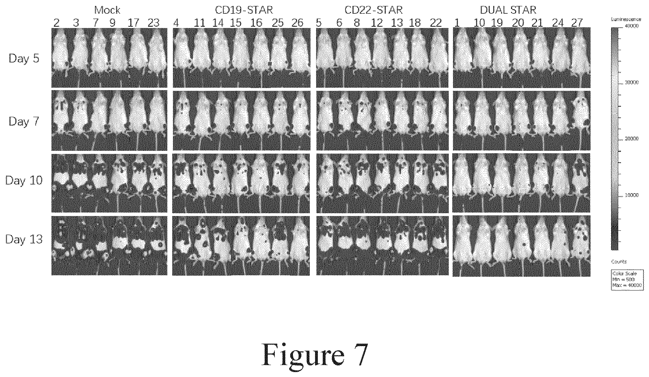

NSG mice were injected with single-target and double-target mixed target cells (90%Raji+5%Raji-CD19KO+5%Raji-CD22KO). 7 days later, mice were respectively injected with CD19-STAR T cells, CD22-STAR T cells and CD19-22 STAR-OX40 T cells, wherein the α chain of these STARs contains the deletion of the intracellular region of the constant region and OX40 co-stimulatory factors through the linker (G4S)₃, and contains the CT modification of the constant region of the α chain and the β chain. The results are shown in Figure 7, injection of single-target STAR-T cannot effectively inhibit tumor growth, while injection of CD19-22 STAR-OX40-T can effectively eliminate tumor cells, indicating that CD19-22 STAR-OX40 T can effectively kill CD19 /22 target cells in vivo.

Sequence listing:
SEQ ID NO:1        Human T cell receptor alpha chain constant region
DIQNPDPAVYQLRDSKSSDKSVCLFTDFDSQTNVSQSKDSDVYITDKTVLDMRSMDFKSNSAVAWSN
KSDFACANAFNNSIIPEDTFFPSPESSCDVKLVEKSFETDTNLNFQNLSVIGFRILLLKVAGFNLLMTLRL
WSS

SEQ ID NO:2        mouse T cell receptor alpha chain constant region
DIQNPEPAVYQLKDPRSQDSTLCLFTDFDSQINVPKTMESGTFITDKTVLDMKAMDSKSNGAIAWSNQ
TSFTCQDIFKETNATYPSSDVPCDATLTEKSFETDMNLNFQNLSVMGLRILLLKVAGFNLLMTLRLWSS

SEQ ID NO:3        mouse T cell receptor alpha chain constant region containing cysteine substitution and
modification of hydrophobic region
DIQNPEPAVYQLKDPRSQDSTLCLFTDFDSQINVPKTMESGTFITDKCVLDMKAMDSKSNGAIAWSNQ
TSFTCQDIFKETNATYPSSDVPCDATLTEKSFETDMNLNFQNLLVIVLRILLLKVAGFNLLMTLRLWSS

SEQ ID NO:4        mouse T cell receptor alpha chain constant region containing N-terminal modification,
cysteine substitution and modification of transmembrane hydrophobic region
DIQNPDPAVYQLRDDSTLCLFTDFDSQINVPKTMESGTFITDKCVLDMKAMDSKSNGAIAWSNQTSFT
CQDIFKETNATYPSSDVPCDATLTEKSFETDMNLNFQNLLVIVLRILLLKVAGFNLLMTLRLWSS

SEQ ID NO:5 Human T cell receptor beta chain constant region
DLKNVFPPEVAVFEPSEAEISHTQKATLVCLATGFYPDHVELSWWVNGKEVHSGVSTDPQPLKEQPAL
NDSRYCLSSRLRVSATFWQNPRNHFRCQVQFYGLSENDEWTQDRAKPVTQIVSAEAWGRADCGFTSE
SYQQGVLSATILYEILLGKATLYAVLVSALVLMAMVKRKDF

SEQ ID NO:6 mouse T cell receptor beta chain constant region
DLRNVTPPKVSLFEPSKAEIANKQKATLVCLARGFFPDHVELSWWVNGKEVHSGVSTDPQAYKESNY
SYCLSSRLRVSATFWHNPRNHFRCQVQFHGLSEEDKWPEGSPKPVTQNISAEAWGRADCGITSASYQQ
GVLSATILYEILLGKATLYAVLVSTLVVMAMVKRKNS

SEQ ID NO:7 mouse T cell receptor beta chain constant region containing cysteine substitution
DLRNVTPPKVSLFEPSKAEIANKQKATLVCLARGFFPDHVELSWWVNGKEVHSGVCTDPQAYKESNY
SYCLSSRLRVSATFWHNPRNHFRCQVQFHGLSEEDKWPEGSPKPVTQNISAEAWGRADCGITSASYQQ
GVLSATILYEILLGKATLYAVLVSTLVVMAMVKRKNS

SEQ ID NO:8 mouse T cell receptor beta chain constant region containing N-terminal modification, and
cysteine substitution
DLKNVFPPEVAVFEPSAEIATLVCLARGFFPDHVELSWWVNGKEVHSGVCTDPQAYKESNYSYCLSSR
LRVSATFWHNPRNHFRCQVQFHGLSEEDKWPEGSPKPVTQNISAEAWGRADCGITSASYQQGVLSATI
LYEILLGKATLYAVLVSTLVVMAMVKRKNS

SEQ ID NO:9 Anti-CD19 FMC63 VH
EVKLQESGPGLVAPSQSLSVTCTVSGVSLPDYGVSWIRQPPRKGLEWLGVIWGSETTYYNSALKSRLTI
IKDNSKSQVFLKMNSLQTDDTAIYYCAKHYYYGGSYAMDYWGQGTSVTVSS

SEQ ID NO:10 Anti-CD19 FMC63 VL
DIQMTQTTSSLSASLGDRVTISCRASQDISKYLNWYQQKPDGTVKLLIYHTSRLHSGVPSRFSGSGSGT
DYSLTISNLEQEDIATYFCQQGNTLPYTFGGGTKLEIT

SEQ ID NO:11 Anti-CD22 M971 VH
QVQLQQSGPGLVKPSQTLSLTCAISGDSVSSNSAAWNWIRQSPSRGLEWLGRTYYRSKWYNDYAVSV
KSRITINPDTSKNQFSLQLNSVTPEDTAVYYCAREVTGDLEDAFDIWGQGTMVTVSS

SEQ ID NO:12 Anti-CD22 M971 VL
DIQMTQSPSSLSASVGDRVTITCRASQTIWSYLNWYQQRPGKAPNLLIYAASSLQSGVPSRFSGRGSGT
DFTLTISSLQAEDFATYYCQQSYSIPQTFGQGTKLEIK

SEQ ID NO:13 CD8 hinge region amino acid sequence
SHFVPVFLPAKPTTTPAPRPPTPAPTIASQPLSLRPEACRPAAGGAVHTRGLDFACD

SEQ ID NO:14 OX40 endodomain
RRDQRLPPDAHKPPGGGSFRTPIQEEQADAHSTLAKI

SEQ ID NO:15 (G4S)3 linker
GGGGSGGGGSGGGGS

SEQ ID NO:16 CD19-22 STAR(CT) α chain
DIQMTQTTSSLSASLGDRVTISCRASQDISKYLNWYQQKPDGTVKLLIYHTSRLHSGVPSRFSGSGSGT
DYSLTISNLEQEDIATYFCQQGNTLPYTFGGGTKLEITGGGGSGGGGSGGGGSEVKLQESGPGLVAPSQ
SLSVTCTVSGVSLPDYGVSWIRQPPRKGLEWLGVIWGSETTYYNSALKSRLTIIKDNSKSQVFLKMNS
LQTDDTAIYYCAKHYYYGGSYAMDYWGQGTSVTVSSDIQNPEPAVYQLKDPRSQDSTLCLFTDFDSQI
NVPKTMESGTFITDKCVLDMKAMDSKSNGAIAWSNQTSFTCQDIFKETNATYPSSDVPCDATLTEKSF
ETDMNLNFQNLLVIVLRILLLKVAGFNLLMTLRLWSS

SEQ ID NO:17 CD19-22 STAR(CT) β chain
DIQMTQSPSSLSASVGDRVTITCRASQTIWSYLNWYQQRPGKAPNLLIYAASSLQSGVPSRFSGRGSGT
DFTLTISSLQAEDFATYYCQQSYSIPQTFGQGTKLEIKGGGGSGGGGSGGGGSQVQLQQSGPGLVKPSQ

TLSLTCAISGDSVSSNSAAWNWIRQSPSRGLEWLGRTYYRSKWYNDYAVSVKSRITINPDTSKNQFSLQ
LNSVTPEDTAVYYCAREVTGDLEDAFDIWGQGTMVTVSSEFSHFVPVFLPAKPTTTPAPRPPTPAPTIAS
QPLSLRPEACRPAAGGAVHTRGLDFACDEDLRNVTPPKVSLFEPSKAEIANKQKATLVCLARGFFPDH
VELSWWVNGKEVHSGVCTDPQAYKESNYSYCLSSRLRVSATFWHNPRNHFRCQVQFHGLSEEDKWP
EGSPKPVTQNISAEAWGRADCGITSASYQQGVLSATILYEILLGKATLYAVLVSTLVVMAMVKRKNS

SEQ ID NO:18    CD19-22 STAR-OX40 α chain(CT)
DIQMTQTTSSLSASLGDRVTISCRASQDISKYLNWYQQKPDGTVKLLIYHTSRLHSGVPSRFSGSGSGT
DYSLTISNLEQEDIATYFCQQGNTLPYTFGGGTKLEITGGGGSGGGGSGGGGSEVKLQESGPGLVAPSQ
SLSVTCTVSGVSLPDYGVSWIRQPPRKGLEWLGVIWGSETTYYNSALKSRLTIIKDNSKSQVFLKMNS
LQTDDTAIYYCAKHYYYGGSYAMDYWGQGTSVTVSSDIQNPEPAVYQLKDPRSQDSTLCLFTDFDSQI
NVPKTMESGTFITDKCVLDMKAMDSKSNGAIAWSNQTSFTCQDIFKETNATYPSSDVPCDATLTEKSF
ETDMNLNFQNLLVIVLRILLLKVAGFNLLMTLRLWGGGGSGGGGSGGGGSRRDQRLPPDAHKPPGGG
SFRTPIQEEQADAHSTLAKI

SEQ ID NO:19    CD19-22 STAR-OX40 β chain(CT)
DIQMTQSPSSLSASVGDRVTITCRASQTIWSYLNWYQQRPGKAPNLLIYAASSLQSGVPSRFSGRGSGT
DFTLTISSLQAEDFATYYCQQSYSIPQTFGQGTKLEIKGGGGSGGGGSGGGGSQVQLQQSGPGLVKPSQ
TLSLTCAISGDSVSSNSAAWNWIRQSPSRGLEWLGRTYYRSKWYNDYAVSVKSRITINPDTSKNQFSLQ
LNSVTPEDTAVYYCAREVTGDLEDAFDIWGQGTMVTVSSEFSHFVPVFLPAKPTTTPAPRPPTPAPTIAS
QPLSLRPEACRPAAGGAVHTRGLDFACDEDLRNVTPPKVSLFEPSKAEIANKQKATLVCLARGFFPDH
VELSWWVNGKEVHSGVCTDPQAYKESNYSYCLSSRLRVSATFWHNPRNHFRCQVQFHGLSEEDKWP
EGSPKPVTQNISAEAWGRADCGITSASYQQGVLSATILYEILLGKATLYAVLVSTLVVMAMVKRKNS

SEQ ID NO:20    CD19-STAR(CT) β chain
DIQMTQTTSSLSASLGDRVTISCRASQDISKYLNWYQQKPDGTVKLLIYHTSRLHSGVPSRFSGSGSGT
DYSLTISNLEQEDIATYFCQQGNTLPYTFGGGTKLEITGGGGSGGGGSGGGGSEVKLQESGPGLVAPSQ
SLSVTCTVSGVSLPDYGVSWIRQPPRKGLEWLGVIWGSETTYYNSALKSRLTIIKDNSKSQVFLKMNS
LQTDDTAIYYCAKHYYYGGSYAMDYWGQGTSVTVSSEDLRNVTPPKVSLFEPSKAEIANKQKATLVC
LARGFFPDHVELSWWVNGKEVHSGVCTDPQAYKESNYSYCLSSRLRVSATFWHNPRNHFRCQVQFH
GLSEEDKWPEGSPKPVTQNISAEAWGRADCGITSASYQQGVLSATILYEILLGKATLYAVLVSTLVVMA
MVKRKNS

SEQ ID NO:21    α chain of CD19-STAR(CT ) or CD22-STAR(CT)
DIQNPEPAVYQLKDPRSQDSTLCLFTDFDSQINVPKTMESGTFITDKCVLDMKAMDSKSNGAIAWSNQ
TSFTCQDIFKETNATYPSSDVPCDATLTEKSFETDMNLNFQNLLVIVLRILLLKVAGFNLLMTLRLWSS*

SEQ ID NO:22    β chain of CD22-STAR(CT) or M971(CD8h)ba
DIQMTQSPSSLSASVGDRVTITCRASQTIWSYLNWYQQRPGKAPNLLIYAASSLQSGVPSRFSGRGSGT
DFTLTISSLQAEDFATYYCQQSYSIPQTFGQGTKLEIKGGGGSGGGGSGGGGSQVQLQQSGPGLVKPSQ
TLSLTCAISGDSVSSNSAAWNWIRQSPSRGLEWLGRTYYRSKWYNDYAVSVKSRITINPDTSKNQFSLQ
LNSVTPEDTAVYYCAREVTGDLEDAFDIWGQGTMVTVSSEFSHFVPVFLPAKPTTTPAPRPPTPAPTIAS
QPLSLRPEACRPAAGGAVHTRGLDFACDEDLRNVTPPKVSLFEPSKAEIANKQKATLVCLARGFFPDH
VELSWWVNGKEVHSGVCTDPQAYKESNYSYCLSSRLRVSATFWHNPRNHFRCQVQFHGLSEEDKWP
EGSPKPVTQNISAEAWGRADCGITSASYQQGVLSATILYEILLGKATLYAVLVSTLVVMAMVKRKNS

SEQ ID NO:23    CD22-STAR(CT) α chain
DIQNPEPAVYQLKDPRSQDSTLCLFTDFDSQINVPKTMESGTFITDKCVLDMKAMDSKSNGAIAWSNQ
TSFTCQDIFKETNATYPSSDVPCDATLTEKSFETDMNLNFQNLLVIVLRILLLKVAGFNLLMTLRLWSS

SEQ ID NO:24    CD19-22 CAR
DIQMTQTTSSLSASLGDRVTISCRASQDISKYLNWYQQKPDGTVKLLIYHTSRLHSGVPSRFSGSGSGT
DYSLTISNLEQEDIATYFCQQGNTLPYTFGGGTKLEITGGGGSQVQLQQSGPGLVKPSQTLSLTCAISGD
SVSSNSAAWNWIRQSPSRGLEWLGRTYYRSKWYNDYAVSVKSRITINPDTSKNQFSLQLNSVTPEDTA
VYYCAREVTGDLEDAFDIWGQGTMVTVSSGSTSGSGKPGSGEGSTKGDIQMTQSPSSLSASVGDRVTI
TCRASQTIWSYLNWYQQRPGKAPNLLIYAASSLQSGVPSRFSGRGSGTDFTLTISSLQAEDFATYYCQQ
SYSIPQTFGQGTKLEIKGGGGSEVKLQESGPGLVAPSQSLSVTCTVSGVSLPDYGVSWIRQPPRKGLEW
LGVIWGSETTYYNSALKSRLTIIKDNSKSQVFLKMNSLQTDDTAIYYCAKHYYYGGSYAMDYWGQG
TSVTVSSSGTTTPAPRPPTPAPTIASQPLSLRPEACRPAAGGAVHTRGLDFACDIYIWAPLAGTCGVLLLS

LVITLYCKRGRKKLLYIFKQPFMRPVQTTQEEDGCSCRFPEEEEGGCELRVKFSRSADAPAYQQGQNQL
YNELNLGRREEYDVLDKRRGRDPEMGGKPRRKNPQEGLYNELQKDKMAEAYSEIGMKGERRRGKG
HDGLYQGLSTATKDTYDALHMQALPPR

SEQ ID NO:25 FMC63 heavy chain variable region CDR1 amino acid sequence
GVSLPDYG

SEQ ID NO:26 FMC63 heavy chain variable region CDR2 amino acid sequence
IWGSETT

SEQ ID NO:27 FMC63 heavy chain variable region CDR3 amino acid sequence
AK

SEQ ID NO:28 FMC63 light chain variable region CDR1 amino acid sequence
QDISKY

SEQ ID NO:29 FMC63 light chain variable region CDR2 amino acid sequence
HTS

SEQ ID NO:30 FMC63 light chain variable region CDR3 amino acid sequence
QQGNTLP

SEQ ID NO:31 M971 heavy chain variable region CDR1 amino acid sequence
GDSVSSNSAA

SEQ ID NO:32 M971 heavy chain variable region CDR2 amino acid sequence
TYYRSKWYN

SEQ ID NO:33 M971 heavy chain variable region CDR3 amino acid sequence
AR

SEQ ID NO:34 M971 light chain variable region CDR1 amino acid sequence
QTIWSY

SEQ ID NO:35 M971 light chain variable region CDR2 amino acid sequence
AAS

SEQ ID NO:36 M971 light chain variable region CDR3 amino acid sequence
QQSYSIP

SEQ ID NO:37 CD19-22 STAR(NCT) α chain
DIQMTQTTSSLSASLGDRVTISCRASQDISKYLNWYQQKPDGTVKLLIYHTSRLHSGVPSRFSGSGSGT
DYSLTISNLEQEDIATYFCQQGNTLPYTFGGGTKLEITGGGGSGGGGSGGGGSEVKLQESGPGLVAPSQ
SLSVTCTVSGVSLPDYGVSWIRQPPRKGLEWLGVIWGSETTYYNSALKSRLTIIKDNSKSQVFLKMNS
LQTDDTAIYYCAKHYYYGGSYAMDYWGQGTSVTVSSDIQNPDPAVYQLRDDSTLCLFTDFDSQINVP
KTMESGTFITDKCVLDMKAMDSKSNGAIAWSNQTSFTCQDIFKETNATYPSSDVPCDATLTEKSFETD
MNLNFQNLLVIVLRILLLKVAGFNLLMTLRLWSS

SEQ ID NO:38 CD19-22 STAR(NCT) β chain
DIQMTQSPSSLSASVGDRVTITCRASQTIWSYLNWYQQRPGKAPNLLIYAASSLQSGVPSRFSGRGSGT
DFTLTISSLQAEDFATYYCQQSYSIPQTFGQGTKLEIKGGGGSGGGGSGGGGSQVQLQQSGPGLVKPSQ
TLSLTCAISGDSVSSNSAAWNWIRQSPSRGLEWLGRTYYRSKWYNDYAVSVKSRITINPDTSKNQFSLQ
LNSVTPEDTAVYYCAREVTGDLEDAFDIWGQGTMVTVSSEFSHFVPVFLPAKPTTTPAPRPPTPAPTIAS
QPLSLRPEACRPAAGGAVHTRGLDFACDEDLKNVFPPEVAVFEPSAEIATLVCLARGFFPDHVELSWW
VNGKEVHSGVCTDPQAYKESNYSYCLSSRLRVSATFWHNPRNHFRCQVQFHGLSEEDKWPEGSPKPV
TQNISAEAWGRADCGITSASYQQGVLSATILYEILLGKATLYAVLVSTLVVMAMVKRKNS

SEQ ID NO:39 Endodomain-deleted mouse T cell receptor α chain constant region

DIQNPEPAVYQLKDPRSQDSTLCLFTDFDSQINVPKTMESGTFITDKTVLDMKAMDSKSNGAIAWSNQ
TSFTCQDIFKETNATYPSSDVPCDATLTEKSFETDMNLNFQNLSVMGLRILLLKVAGFNLLMTLRLW

SEQ ID NO:40 Endodomain-deleted mouse T cell receptor α chain constant region with cysteine substitution and hydrophobic modification
DIQNPEPAVYQLKDPRSQDSTLCLFTDFDSQINVPKTMESGTFITDKCVLDMKAMDSKSNGAIAWSNQ
TSFTCQDIFKETNATYPSSDVPCDATLTEKSFETDMNLNFQNLLVIVLRILLLKVAGFNLLMTLRLW

SEQ ID NO:41 Endodomain-deleted mouse T cell receptor α chain constant region with N-terminal modification, cysteine substitution and modification of transmembrane hydrophobic region
DIQNPDPAVYQLRDDSTLCLFTDFDSQINVPKTMESGTFITDKCVLDMKAMDSKSNGAIAWSNQTSFT
CQDIFKETNATYPSSDVPCDATLTEKSFETDMNLNFQNLLVIVLRILLLKVAGFNLLMTLRLW

SEQ ID NO:42 Endodomain-deleted mouse T cell receptor β chain constant region
DLRNVTPPKVSLFEPSKAEIANKQKATLVCLARGFFPDHVELSWWVNGKEVHSGVSTDPQAYKESNY
SYCLSSRLRVSATFWHNPRNHFRCQVQFHGLSEEDKWPEGSPKPVTQNISAEAWGRADCGITSASYQQ
GVLSATILYEILLGKATLYAVLVSTLVVMAM

SEQ ID NO:43 Endodomain-deleted mouse T cell receptor β chain constant region with cysteine substitution
DLRNVTPPKVSLFEPSKAEIANKQKATLVCLARGFFPDHVELSWWVNGKEVHSGVCTDPQAYKESNY
SYCLSSRLRVSATFWHNPRNHFRCQVQFHGLSEEDKWPEGSPKPVTQNISAEAWGRADCGITSASYQQ
GVLSATILYEILLGKATLYAVLVSTLVVMAM

SEQ ID NO:44 Endodomain-deleted mouse T cell receptor β chain constant region with N-terminal modification and cysteine substitution
DLKNVFPPEVAVFEPSAEIATLVCLARGFFPDHVELSWWVNGKEVHSGVCTDPQAYKESNYSYCLSSR
LRVSATFWHNPRNHFRCQVQFHGLSEEDKWPEGSPKPVTQNISAEAWGRADCGITSASYQQGVLSATI
LYEILLGKATLYAVLVSTLVVMAM

SEQ ID NO:45 Anti-CD22 HA22 VL
DIQMTQTTSSLSASLGDRVTISCRASQDISNYLNWYQQKPDGTVKLLIYYTSILHSGVPSRFSGSGSGT
DYSLTISNLEQEDFATYFCQQGNTLPWTFGGGTKLEIKA

SEQ ID NO:46 Anti-CD22 HA22 VH
EVQLVESGGGLVKPGGSLKLSCAASGFAFSIYDMSWVRQTPEKRLEWVAYISSGGGTYYPDTVKGRFT
ISRDNAKNTLYLQMSSLKSEDTAMYYCARHSGYGSSYGVLFAYWGQGTLVTVSA

SEQ ID NO:47 HA22-FMC63 dual-target STAR β chain
DIQMTQTTSSLSASLGDRVTISCRASQDISNYLNWYQQKPDGTVKLLIYYTSILHSGVPSRFSGSGSGT
DYSLTISNLEQEDFATYFCQQGNTLPWTFGGGTKLEIKAGGGGSGGGGSGGGGSEVQLVESGGGLVKP
GGSLKLSCAASGFAFSIYDMSWVRQTPEKRLEWVAYISSGGGTYYPDTVKGRFTISRDNAKNTLYLQ
MSSLKSEDTAMYYCARHSGYGSSYGVLFAYWGQGTLVTVSAEDLRNVTPPKVSLFEPSKAEIANKQK
ATLVCLARGFFPDHVELSWWVNGKEVHSGVCTDPQAYKESNYSYCLSSRLRVSATFWHNPRNHFRCQ
VQFHGLSEEDKWPEGSPKPVTQNISAEAWGRADCGITSASYQQGVLSATILYEILLGKATLYAVLVSTL
VVMAMVKRKNS

SEQ ID NO:48 HA22-FMC63 dual-target STAR α chain
DIQMTQTTSSLSASLGDRVTISCRASQDISKYLNWYQQKPDGTVKLLIYHTSRLHSGVPSRFSGSGSGT
DYSLTISNLEQEDIATYFCQQGNTLPYTFGGGTKLEITGGGGSGGGGSGGGGSEVKLQESGPGLVAPSQ
SLSVTCTVSGVSLPDYGVSWIRQPPRKGLEWLGVIWGSETTYYNSALKSRLTIIKDNSKSQVFLKMNS
LQTDDTAIYYCAKHYYYGGSYAMDYWGQGTSVTVSSDIQNPEPAVYQLKDPRSQDSTLCLFTDFDSQI
NVPKTMESGTFITDKCVLDMKAMDSKSNGAIAWSNQTSFTCQDIFKETNATYPSSDVPCDATLTEKSF
ETDMNLNFQNLLVIVLRILLLKVAGFNLLMTLRLWGGGGSGGGGSGGGGSRRDQRLPPDAHKPPGGG
SFRTPIQEEQADAHSTLAKI

SEQ ID NO:49 HA22(M218)-FMC63 dual-target STAR β chain
DIQMTQTTSSLSASLGDRVTISCRASQDISNYLNWYQQKPDGTVKLLIYYTSILHSGVPSRFSGSGSGT

DYSLTISNLEQEDFATYFCQQGNTLPWTFGGGTKLEIKAGSTSGSGKPGSGEGSTKGEVQLVESGGGLV
KPGGSLKLSCAASGFAFSIYDMSWVRQTPEKRLEWVAYISSGGGTYYPDTVKGRFTISRDNAKNTLYL
QMSSLKSEDTAMYYCARHSGYGSSYGVLFAYWGQGTLVTVSAEDLRNVTPPKVSLFEPSKAEIANKQ
KATLVCLARGFFPDHVELSWWVNGKEVHSGVCTDPQAYKESNYSYCLSSRLRVSATFWHNPRNHFRC
QVQFHGLSEEDKWPEGSPKPVTQNISAEAWGRADCGITSASYQQGVLSATILYEILLGKATLYAVLVST
LVVMAMVKRKNS

SEQ ID NO:50 HA22(M218)-FMC63 dual-target STAR α chain
DIQMTQTTSSLSASLGDRVTISCRASQDISKYLNWYQQKPDGTVKLLIYHTSRLHSGVPSRFSGSGSGT
DYSLTISNLEQEDIATYFCQQGNTLPYTFGGGTKLEITGGGGSGGGGSGGGGSEVKLQESGPGLVAPSQ
SLSVTCTVSGVSLPDYGVSWIRQPPRKGLEWLGVIWGSETTYYNSALKSRLTIIKDNSKSQVFLKMNS
LQTDDTAIYYCAKHYYYGGSYAMDYWGQGTSVTVSSDIQNPEPAVYQLKDPRSQDSTLCLFTDFDSQI
NVPKTMESGTFITDKCVLDMKAMDSKSNGAIAWSNQTSFTCQDIFKETNATYPSSDVPCDATLTEKSF
ETDMNLNFQNLLVIVLRILLLKVAGFNLLMTLRLWGGGGSGGGGSGGGGSRRDQRLPPDAHKPPGGG
SFRTPIQEEQADAHSTLAKI

SEQ ID NO:51 HA22(4G4S)-FMC63 dual-target STAR β chain
DIQMTQTTSSLSASLGDRVTISCRASQDISNYLNWYQQKPDGTVKLLIYYTSILHSGVPSRFSGSGSGT
DYSLTISNLEQEDFATYFCQQGNTLPWTFGGGTKLEIKAGGGGSGGGGSGGGGSGGGGSEVQLVESG
GGLVKPGGSLKLSCAASGFAFSIYDMSWVRQTPEKRLEWVAYISSGGGTYYPDTVKGRFTISRDNAKN
TLYLQMSSLKSEDTAMYYCARHSGYGSSYGVLFAYWGQGTLVTVSAEDLRNVTPPKVSLFEPSKAEI
ANKQKATLVCLARGFFPDHVELSWWVNGKEVHSGVCTDPQAYKESNYSYCLSSRLRVSATFWHNPR
NHFRCQVQFHGLSEEDKWPEGSPKPVTQNISAEAWGRADCGITSASYQQGVLSATILYEILLGKATLYA
VLVSTLVVMAMVKRKNS

SEQ ID NO:52 HA22(4G4S)-FMC63 dual-target STAR α chain
DIQMTQTTSSLSASLGDRVTISCRASQDISKYLNWYQQKPDGTVKLLIYHTSRLHSGVPSRFSGSGSGT
DYSLTISNLEQEDIATYFCQQGNTLPYTFGGGTKLEITGGGGSGGGGSGGGGSEVKLQESGPGLVAPSQ
SLSVTCTVSGVSLPDYGVSWIRQPPRKGLEWLGVIWGSETTYYNSALKSRLTIIKDNSKSQVFLKMNS
LQTDDTAIYYCAKHYYYGGSYAMDYWGQGTSVTVSSDIQNPEPAVYQLKDPRSQDSTLCLFTDFDSQI
NVPKTMESGTFITDKCVLDMKAMDSKSNGAIAWSNQTSFTCQDIFKETNATYPSSDVPCDATLTEKSF
ETDMNLNFQNLLVIVLRILLLKVAGFNLLMTLRLWGGGGSGGGGSGGGGSRRDQRLPPDAHKPPGGG
SFRTPIQEEQADAHSTLAKI

SEQ ID NO:53 HA22(CD8h)-FMC63 dual-target STAR β chain
DIQMTQTTSSLSASLGDRVTISCRASQDISNYLNWYQQKPDGTVKLLIYYTSILHSGVPSRFSGSGSGT
DYSLTISNLEQEDFATYFCQQGNTLPWTFGGGTKLEIKAGGGGSGGGGSGGGGSEVQLVESGGGLVKP
GGSLKLSCAASGFAFSIYDMSWVRQTPEKRLEWVAYISSGGGTYYPDTVKGRFTISRDNAKNTLYLQ
MSSLKSEDTAMYYCARHSGYGSSYGVLFAYWGQGTLVTVSAEFSHFVPVFLPAKPTTTPAPRPPTPAP
TIASQPLSLRPEACRPAAGGAVHTRGLDFACDEDLRNVTPPKVSLFEPSKAEIANKQKATLVCLARGFF
PDHVELSWWVNGKEVHSGVCTDPQAYKESNYSYCLSSRLRVSATFWHNPRNHFRCQVQFHGLSEED
KWPEGSPKPVTQNISAEAWGRADCGITSASYQQGVLSATILYEILLGKATLYAVLVSTLVVMAMVKRK
NS

SEQ ID NO:54 HA22(CD8h)-FMC63 dual-target STAR α chain
DIQMTQTTSSLSASLGDRVTISCRASQDISKYLNWYQQKPDGTVKLLIYHTSRLHSGVPSRFSGSGSGT
DYSLTISNLEQEDIATYFCQQGNTLPYTFGGGTKLEITGGGGSGGGGSGGGGSEVKLQESGPGLVAPSQ
SLSVTCTVSGVSLPDYGVSWIRQPPRKGLEWLGVIWGSETTYYNSALKSRLTIIKDNSKSQVFLKMNS
LQTDDTAIYYCAKHYYYGGSYAMDYWGQGTSVTVSSDIQNPEPAVYQLKDPRSQDSTLCLFTDFDSQI
NVPKTMESGTFITDKCVLDMKAMDSKSNGAIAWSNQTSFTCQDIFKETNATYPSSDVPCDATLTEKSF
ETDMNLNFQNLLVIVLRILLLKVAGFNLLMTLRLWGGGGSGGGGSGGGGSRRDQRLPPDAHKPPGGG
SFRTPIQEEQADAHSTLAKI

SEQ ID NO:55 HA22VL-CDR1
QDISNY

SEQ ID NO:56 HA22VL-CDR2

YTS

SEQ ID NO:57 HA22VL-CDR3
QQGNTLP

SEQ ID NO:58 HA22VH-CDR1
GFAFSIYD

SEQ ID NO:59 HA22VH-CDR2
ISSGGGT

SEQ ID NO:60 HA22VH-CDR3
AR

SEQ ID NO:61 BL22VL-CDR1
QDISNY

SEQ ID NO:62 BL22VL-CDR2
YTS

SEQ ID NO:63 BL22VL-CDR3
QQGNTLP

SEQ ID NO:64 BL22VL
DIQMTQTTSSLSASLGDRVTISCRASQDISNYLNWYQQKPDGTVKLLIYYTSILHSGVPSRFSGSGSGT
DYSLTISNLEQEDFATYFCQQGNTLPWTFGGGTKLEIKA

SEQ ID NO:65 BL22VH-CDR1
GFAFSIYD

SEQ ID NO:66 BL22VH-CDR2
ISSGGGT

SEQ ID NO:67 BL22VH-CDR3
AR

SEQ ID NO:68 BL22VH
EVQLVESGGGLVKPGGSLKLSCAASGFAFSIYDMSWVRQTPEKRLEWVAYISSGGGTYYPDTVKGRFT
ISRDNAKNTLYLQMSSLKSEDTAMYYCARHSGYGTHWGVLFAYWGQGTLVTVSA

SEQ ID NO:69 M972VL-CDR1
QSFSNM

SEQ ID NO:70 M972VL-CDR2
GVS

SEQ ID NO:71 M972VL-CDR3
QQYGDWP

SEQ ID NO:72 M972VL
RIVMTQSPGTLSVSPGETATLSCRASQSFSNMLAWYQQKSGQPPRLLIYGVSTRAAGVPARFSGSGSGT
EFTLTISNLQSEDFAVYYCQQYGDWPRYTFGQGTKVERK

SEQ ID NO:73 M972VH-CDR1
GFTFDDYG

SEQ ID NO:74 M972VH-CDR2
INWNGGST

SEQ ID NO:75 M972VH-CDR3
AR

SEQ ID NO:76 M972VH
VQLVQSGGGVVRPGGSLRLPCAASGFTFDDYGMSWVRQAPGKGLEWVSGINWNGGSTGYADSVKG
RFTISRDNAKNSLYLQMNSLRAEDTALYHCARGGDYDAFDIWGQGTMVTVSS

SEQ ID NO:77 HRFB4VL-CDR1
QDISNY

SEQ ID NO:78 HRFB4VL-CDR2
YTS

SEQ ID NO:79 HRFB4VL-CDR3
QQGNTLP

SEQ ID NO:80 HRFB4VL
DIQLTQSPSSLSASVGDRVTISCRASQDISNYLNWYQQKPGKAPKLLIYYTSILHSGVPSRFSGSGSGTD
YTFTISSLQPEDIATYYCQQGNTLPWTFGGGTKVQIKR

SEQ ID NO:81 HRFB4VH-CDR1
GFTFSIYD

SEQ ID NO:82 HRFB4VH-CDR2
ISSGGGTT

SEQ ID NO:83 HRFB4VH-CDR3
AR

SEQ ID NO:84 HRFB4VH
QVQLVQSGAEVKKPGSSVKVSCKASGFTFSIYDMSWVRQAPGQGLEWVAYISSGGGTTYYPDTVKGR
FTITADESTNTAYMELSSLRSEDTAFYFCARHSGYGSSYGVLFAYWGQGTLVTVSS

## Claims

1. A synthetic T cell receptor and antigen receptor (STAR) against CD19 and CD22, which comprises an α chain comprising a first antigen binding region and a first constant region, and a β chain comprising a second antigen binding region and a second constant region,
wherein the first antigen binding region is an antigen binding region specifically binding to CD19, and the second antigen binding region is an antigen binding region specifically binding to CD22; or the first antigen binding region is an antigen binding region specifically binding to CD22, and the second antigen binding region is an antigen binding region specifically binding to CD 19.

2. The STAR against CD19 and CD22 according to claim 1, wherein the first constant region is a modified TCR α chain constant region.

3. The STAR against CD19 and CD22 according to claim 2, wherein the modified TCRα chain constant region is derived from a mouse TCRα chain constant region, and the amino acid at position 48, such as threonine T, is mutated to cysteine C, as compared to the wild-type mouse TCRα chain constant region.

4. The STAR against CD19 and CD22 according to claim 2 or 3, wherein the modified TCR α chain constant region is derived from a mouse TCR α chain constant region, the amino acid at position 112 such as serine S is mutated to leucine L, the amino acid at position 114 such as methionine M is mutated to isoleucine I, and the amino acid at position 115 such as glycine G is mutated to valine V, as compared to the wild-type mouse TCRα chain constant region.

5. The STAR against CD19 and CD22 according to any one of claims 2-4, wherein the modified TCRα chain constant region is derived from a mouse TCRα chain constant region, the amino acid at position 48, such as threonine T, is mutated to Cysteine C, the amino acid at position 112 such as serine S is mutated to leucine L, the amino acid at position 114 such as methionine M is mutated to isoleucine I, the amino acid at position 115 such as Glycine G is mutated to valine V, as compared to the wild-type mouse TCRα chain constant region.

6. The STAR against CD19 and CD22 according to any one of claims 2-5, wherein the modified TCR α chain constant region is derived from the mouse TCR α chain constant region, the amino acid at position 6 such as E is substituted by D, and the amino acid K at position 13 is substituted by R, and amino acids at positions 15-18 are deleted, as compared to the wild-type mouse TCRα chain constant region.

7. The STAR against CD19 and CD22 according to any one of claims 2-6, wherein the modified TCRα chain constant region is derived from the mouse TCRα chain constant region, the amino acid at position 6, such as E, is substituted by D, and the amino acid K at position 13 is substituted by R, the amino acids at positions 15-18 are deleted, the amino acid at position 48 such as threonine T is mutated to cysteine C, and the amino acid at position 112 such as serine S is mutated to leucine acid L, the amino acid at position 114 such as methionine M is mutated to isoleucine I, and the amino acid at position 115 such as glycine G is mutated to valine V, as compared to the wild-type mouse TCRα chain constant region.

8. The STAR against CD19 and CD22 according to any one of claims 2-6, wherein the modified TCR α chain constant region is derived from the mouse TCR α chain constant region, and lacks the endodomain of the constant region, for example, the amino acids at positions 136-137 are deleted, as compared to the wild-type mouse TCRα chain constant region.

9. The STAR against CD19 and CD22 according to claim 1, wherein the modified TCRα chain constant region comprises an amino acid sequence shown in one of SEQ ID NOs: 3-4 and 39-41.

10. The STAR against CD19 and CD22 according to any one of claims 1-9, wherein the second constant region is a modified TCR β chain constant region.

11. The STAR against CD19 and CD22 according to claim 10, wherein the modified TCR β chain constant region is derived from the mouse TCR β chain constant region, and the amino acid at position 56, such as serine S, is mutated to cysteine C, as compared to the wild-type mouse TCRβ chain constant region.

12. The STAR against CD19 and CD22 according to any one of claims 10-11, wherein the modified TCR β chain constant region is derived from the mouse TCR β chain constant region, the amino acid at position 3 such as R is substituted by K, the amino acid at position 6 such as T is substituted by F, the amino acid K at position 9 is substituted by E, S at position 11 is substituted by A, L at position 12 is substituted by V, and amino acids at positions 17 and 21-25 are deleted, as compared to the wild-type mouse TCRβ chain constant region.

13. The STAR against CD19 and CD22 according to any one of claims 10-12, wherein the modified TCR β chain constant region is derived from a mouse TCR β chain constant region, the amino acid at position 56 such as serine S is mutated to cysteine C, the amino acid at position 3 such as R is substituted by K, the amino acid at position 6 such as T is substituted by F, K at position 9 is substituted by E, S at position 11 is substituted by A, and L at position 12 is substituted by V, and amino acids 17, 21-25 are deleted, a as compared to the wild-type mouse TCRβ chain constant region.

14. The STAR against CD19 and CD22 according to any one of claims 10-13, wherein the modified TCR β chain constant region is derived from the mouse TCR beta chain constant region, and lacks the endodomain of the constant region, for example, the amino acids at positions 167-172 are deleted, as compared to the wild-type mouse TCRP chain constant region.

15. The STAR against CD19 and CD22 according to claim 14, wherein the modified TCRβ chain constant region comprises an amino acid sequence shown in one of SEQ ID NOs:7-8 and 42-44.

16. The STAR against CD19 and CD22 according to any one of claims 1-15, wherein the antigen binding region is a single chain antibody (such as scFv) or a single domain antibody (such as a camelid antibody), preferably, the antigen binding region is a single chain antibody such as a scFv.

17. The STAR against CD19 and CD22 according to claim 16, wherein the single chain antibody comprises a heavy chain variable region and a light chain variable region linked by a linker, for example, the linker is a (G4S)n linker, wherein n represents an integer from 1-10, preferably, n is 1 or 3.

18. The STAR against CD19 and CD22 according to any one of claims 1-17, wherein the antigen-binding region that specifically binds to CD19 comprises a heavy chain variable region and a light chain variable region, and the heavy chain variable region comprises VH CDR1 shown in SEQ ID NO:25, VH CDR2 shown in SEQ ID NO:26, and VH CDR3 shown in SEQ ID NO:27, the light chain variable region comprises VL CDR1 shown in SEQ ID NO:28, VL CDR2 shown in SEQ ID NO:29 , and VL CDR3 shown in SEQ ID NO:30,
for example, the antigen-binding region that specifically binds to CD19 comprises a heavy chain variable region shown in SEQ ID NO:9, and a light chain variable region shown in SEQ ID NO:10.

19. The STAR against CD19 and CD22 according to any one of claims 1-18, wherein the antigen-binding region that specifically binds to CD22 comprises a heavy chain variable region and a light chain variable region, wherein
i) the heavy chain variable region comprises VH CDR1 shown in SEQ ID NO: 31, VH CDR2 shown in SEQ ID NO: 32, and VH CDR3 shown in SEQ ID NO:33, the light chain variable region comprises VL CDR1 shown in SEQ ID NO:34, VL CDR2 shown in SEQ ID NO:35 , and VL CDR3 shown in SEQ ID NO:36;
ii) the heavy chain variable region comprises VH CDR1 shown in SEQ ID NO:58, VH CDR2 shown in SEQ ID NO: 59, and VH CDR3 shown in SEQ ID NO:60, the light chain variable region comprises VL CDR1 shown in SEQ ID NO:55, VL CDR2 shown in SEQ ID NO:56 , and VL CDR3 shown in SEQ ID NO:57;
iii) the heavy chain variable region comprises VH CDR1 shown in SEQ ID NO:65, VH CDR2 shown in SEQ ID NO: 66, and VH CDR3 shown in SEQ ID NO:67, said light chain variable region comprises VL CDR1 shown in SEQ ID NO:61, VL CDR2 shown in SEQ ID NO:62 , and VL CDR3 shown in SEQ ID NO:63;
iv) the heavy chain variable region comprises VH CDR1 shown in SEQ ID NO:73, VH CDR2 shown in SEQ ID NO:74, and VH CDR3 shown in SEQ ID NO:75, said light chain variable region comprises VL CDR1 shown in SEQ ID NO:69, VL CDR2 shown in SEQ ID NO:70 , and VL CDR3 shown in SEQ ID NO:71; or
v) the heavy chain variable region comprises VH CDR1 shown in SEQ ID NO:81, VH CDR2 shown in SEQ ID NO: 82, and VH CDR3 shown in SEQ ID NO:83, said light chain variable region comprises VL CDR1 shown in SEQ ID NO:77, VL CDR2 shown in SEQ ID NO:78 , and VL CDR3 shown in SEQ ID NO:79,
for example, the antigen-binding region that specifically binds to CD22 comprises
a) the heavy chain variable region shown in SEQ ID NO: 11, and the light chain variable region shown in SEQ ID NO: 12;
b) the heavy chain variable region shown in SEQ ID NO:46, and the light chain variable region shown in SEQ ID NO:45;
c) the heavy chain variable region shown in SEQ ID NO:68, and the light chain variable region shown in SEQ ID NO:64;
d) the heavy chain variable region shown in SEQ ID NO:76, and the light chain variable region shown in SEQ ID NO72; or
e) The heavy chain variable region shown in SEQ ID NO:84, and the light chain variable region shown in SEQ ID NO80.

20. The STAR against CD19 and CD22 according to any one of claims 1-19, wherein the antigen binding region of the α chain or β chain is fused directly or indirectly to the N-terminus of the constant region.

21. The STAR against CD19 and CD22 according to any one of claims 1-20, wherein the antigen-binding region of the α-chain or β-chain is fused to the N-terminus of the constant region via a linker.

22. The STAR against CD19 and CD22 according to claim 21, wherein the antigen-binding region of the α-chain or β-chain, preferably β-chain, is fused to the N-terminus of the constant region via a CD8 hinge region, for example, the CD8 hinge region comprises the amino acid sequence shown in SEQ ID NO: 13.

23. The STAR against CD19 and CD22 according to any one of claims 1-22, wherein the α chain and/or β chain has at least one exogenous intracellular functional domain linked to its C-terminus.

24. The STAR against CD19 and CD22 according to claim 23, wherein the exogenous intracellular functional domain is linked to the C-terminus of the constant region of the α chain and/or β chain directly or through a linker,
preferably, the exogenous intracellular functional domain is linked to the C-terminus of the constant region of the α chain and/or β chain whose endodomain is deleted through a linker.
preferably, the linker is a (G4S)n linker, where n represents an integer from 1-10, preferably, n is 3.

25. The STAR against CD19 and CD22 according to any one of claims 23-24, wherein the exogenous intracellular functional domain is an endodomain of a co-stimulatory molecule, preferably an endodomain of OX40, for example, the endodomain of OX40 comprises the amino acid sequence of SEQ ID NO:14.

26. The STAR against CD19 and CD22 according to any one of claims 1-25, wherein the α chain comprises an antigen-binding region specifically binding to CD19 and a modified TCR α chain constant region, wherein
the antigen-binding region specifically binding to CD19 comprises a heavy chain variable region shown in SEQ ID NO: 9 and a light chain variable region shown in SEQ ID NO: 10 linked by a linker (G4S)₃,
the modified TCR α chain constant region is derived from the mouse TCR α chain constant region, the amino acid at position 48 such as threonine T is mutated to cysteine C, the amino acid at position 112 such as serine S is mutated to leucine L, the amino acid at position 114 such as methionine M is mutated to isoleucine I, and the amino acid at position 115 such as glycine G is mutated to valine V, as compared to the wild-type mouse TCR α chain constant region.

27. The STAR against CD19 and CD22 according to any one of claims 1-25, wherein the α chain comprises an antigen-binding region specifically binding to CD19 and a modified TCR α chain constant region, wherein
the antigen-binding region specifically binding to CD19 comprises a heavy chain variable region shown in SEQ ID NO: 9 and a light chain variable region shown in SEQ ID NO: 10 linked by a linker (G4S)₃,
the modified TCR α chain constant region is derived from the mouse TCR α chain constant region, the amino acid at position 6 such as E is substituted by D, K at position 13 is substituted by R, the amino acids at positions 15-18 are deleted, and the amino acid at position 48 such as threonine T is mutated to cysteine C, the amino acid at position 112 such as serine S is mutated to leucine L, and the amino acid at position 114 such as methionine M is mutated to isoleucine I, and the amino acid at position 115 such as glycine G is mutated to valine V, as compared to the wild-type mouse TCR α chain constant region.

28. The STAR against CD19 and CD22 according to any one of claims 1-25, wherein the α chain comprises an antigen-binding region specifically binding to CD19, a modified TCR α chain constant region, and an endodomain of OX40, wherein
the antigen-binding region specifically binding to CD19 comprises a heavy chain variable region shown in SEQ ID NO: 9 and a light chain variable region shown in SEQ ID NO: 10 linked by a linker (G4S)₃,
the modified TCR α chain constant region is derived from the mouse TCR α chain constant region, the amino acid at position 6 such as E is substituted by D, K at position 13 is substituted by R, the amino acids at positions 15-18 are deleted, and the amino acid at position 48 such as threonine T is mutated to cysteine C, the amino acid at position 112 such as serine S is mutated to leucine L, the amino acid at position 114 such as methionine M is mutated to isoleucine I, and the amino acid at position 115 such as glycine G is mutated to valine V, and amino acids 136-137 are deleted, as compared to the wild-type mouse TCR α chain constant region.

29. The STAR against CD19 and CD22 according to claim 1, wherein the α chain comprises an amino acid sequence set forth in SEQ ID NO: 16, 18 or 37.

30. The STAR against CD19 and CD22 according to any one of claims 1-27, wherein the β chain comprises an antigen-binding region specifically binding to CD22 and a modified TCR β chain constant region, wherein
the antigen-binding region specifically binding to CD22 comprises a heavy chain variable region shown in SEQ ID NO: 11 and a light chain variable region shown in SEQ ID NO: 12 linked by a linker (G4S)₃,
the modified TCRβ chain constant region is derived from the mouse TCRβ chain constant region, and the amino acid at position 56 such as serine S is mutated to cysteine C, as compared to the wild type mouse TCRP chain constant region.

31. The STAR against CD19 and CD22 according to any one of claims 1-27, wherein the β chain comprises an antigen-binding region specifically binding to CD22 and a modified TCR β chain constant region, wherein
the antigen-binding region specifically binding to CD22 comprises a heavy chain variable region shown in SEQ ID NO: 11 and a light chain variable region shown in SEQ ID NO: 12 linked by a linker (G4S)₃,
the modified TCRβ chain constant region is derived from the mouse TCRβ chain constant region, and the amino acid at position 56 such as serine S is mutated to cysteine C, as compared to the wild type mouse TCRβ chain constant region,
wherein the antigen-binding region specifically binding to CD22 is linked to the modified TCRβ chain constant region through a CD8 hinge region.

32. The STAR against CD19 and CD22 according to any one of claims 1-27, wherein the β chain comprises an antigen-binding region specifically binding to CD22 and a modified TCR β chain constant region, wherein
the antigen-binding region specifically binding to CD22 comprises a heavy chain variable region shown in SEQ ID NO: 11 and a light chain variable region shown in SEQ ID NO: 12 linked by a linker (G4S)₃,
the modified TCR β chain constant region is derived from the mouse TCR beta chain constant region, the amino acid at position 3, such as R, is substituted by K, and the amino acid at position 6, such as T, is substituted by F, K at position 9 is substituted by E, S at position 11 is substituted by A, L at position 12 is substituted by V, and amino acids at positions 17 and 21-25 are deleted, and the amino acid at position 56, for example, Serine S is mutated to cysteine C, as compared to the wild type mouse TCRβ chain constant region,
wherein the antigen-binding region specifically binding to CD22 is linked to the modified TCRβ chain constant region through a CD8 hinge region.

33. The STAR against CD19 and CD22 according to any one of claims 1-27, wherein the β chain comprises an antigen-binding region specifically binding to CD22, a modified TCR β chain constant region and an endodomain of OX40, wherein
the antigen-binding region specifically binding to CD22 comprises a heavy chain variable region shown in SEQ ID NO: 11 and a light chain variable region shown in SEQ ID NO: 12 linked by a linker (G4S)₃,
the modified TCR β chain constant region is derived from the mouse TCR beta chain constant region, the amino acid at position 3, such as R, is substituted by K, and the amino acid at position 6, such as T, is substituted by F, K at position 9 is substituted by E, S at position 11 is substituted by A, L at position 12 is substituted by V, and amino acids at positions 17 and 21-25 are deleted, and the amino acid at position 56, for example, Serine S is mutated to cysteine C, as compared to the wild type mouse TCRβ chain constant region,
wherein the antigen-binding region specifically binding to CD22 is linked to the modified TCRβ chain constant region through a CD8 hinge region.

34. The STAR against CD19 and CD22 according to claim 1, wherein the β-strand comprises an amino acid sequence set forth in SEQ ID NO: 17, 19 or 38.

35. The STAR against CD19 and CD22 according to claim 1, wherein the α chain comprises the amino acid sequence set forth in SEQ ID NO:16, and the β chain comprises the amino acid sequence set forth in SEQ ID NO:17.

36. The STAR against CD19 and CD22 according to claim 1, wherein the α chain comprises the amino acid sequence set forth in SEQ ID NO:18, and the β chain comprises the amino acid sequence set forth in SEQ ID NO:19.

37. The STAR against CD19 and CD22 according to claim 1, wherein the α chain comprises the amino acid sequence set forth in SEQ ID NO:37, and the β chain comprises the amino acid sequence set forth in SEQ ID NO:38.

38. An isolated polynucleotide comprising a nucleotide sequence encoding the α-chain and/or β-chain of the STAR against CD19 and CD22 according to any one of claims 1-37.

39. The polynucleotide according to claim 38, the polynucleotide comprises i) a nucleotide sequence encoding the α chain, ii) a nucleotide sequence encoding the β chain, and iii) a nucleotide sequence encoding a self-cleavage peptide located between i) and ii), in a same reading frame.

40. The polynucleotide according to claim 39, wherein the self-cleavage peptide is a 2A polypeptide, such as a P2A polypeptide.

41. An expression vector comprising the polynucleotide of any one of claims 38-40 operably linked to a regulatory sequence.

42. The expression vector according to claim 41, which is a viral vector, such as a lentiviral vector.

43. A method of producing a therapeutic T cell comprising expressing in the T cell the STAR against CD19 and CD22 according to any one of claims 1-37.

44. The method according to claim 43, the method comprises introducing the polynucleotide according to any one of claims 38-40 or the expression vector according to claim 41 or 42 into the T cell.

45. A therapeutic T cell which comprises the STAR against CD19 and CD22 according to any one of claims 1-37, or is obtained by the method according to claim 43 or 44.

46. A pharmaceutical composition, which comprises the therapeutic T cell according to claim 45 and a pharmaceutically acceptable carrier.

47. Use of the therapeutic T cell according to claim 45 or the pharmaceutical composition according to claim 46 in the preparation of a medicine for the treatment of a disease in a subject, for example, the disease is a CD19 and/or CD22-related cancer.

48. The use according to claim 47, wherein the cancer is a B-cell malignant tumor, such as chronic or acute leukemia (including acute myeloid leukemia, chronic myeloid leukemia, acute lymphoblastic leukemia, chronic lymphoblastic leukemia), lymphocytic lymphoma, non-Hodgkin's lymphoma, and combinations thereof.
